# EUROPEAN PATENT SPECIFICATION

(11) **EP 2 118 006 B1**
(45) Date of publication and mention of the grant of the patent: **18.07.2012**
(21) Application number: 08718538.5
(22) Date of filing: 22.02.2008
(51) Int. Cl.: C01B 33/152, C01B 33/157, A61K 9/00

(54) **METHOD FOR PREPARING SILICA COMPOSITIONS, SILICA COMPOSITIONS AND USES THEREOF**
VERFAHREN ZUR HERSTELLUNG VON SILICIUMDIOXIDZUSAMMENSETZUNGEN, SILICIUMDIOXIDZUSAMMENSETZUNGEN UND VERWENDUNG DAVON
MÉTHODE DE PRODUCTION DE COMPOSITIONS DE SILICE, COMPOSITIONS DE SILICE ET LEURS UTILISATIONS

(30) Priority: 28.02.2007 FI 20070174; 28.02.2007 US 903824 P
(43) Date of publication of application: 18.11.2009
(60) Divisional application: 12151050.7
(73) Proprietor: DelSiTech Oy, 20520 Turku (FI)
(72) Inventor: JOKINEN, Mika, FI-20540 Turku (FI); JALONEN, Harry, FI-20610 Turku (FI); FORSBACK, Ari-Pekka, FI-20540 Turku (FI); KOSKINEN, Mika, FI-20460 Turku (FI)
(74) Representative: Suominen, Kaisa Liisa
(86) International application number: PCT/FI2008/050085
(87) International publication number: WO 2008/104635

(56) References cited:
- GB-A- 590 902
- GB-A- 2 103 202
- US-A1- 2006 171 990
- CELLESI ET AL: "Sol-gel synthesis at neutral pH in W/O microemulsion: A method for enzyme nanoencapsulation in silica gel nanoparticles" COLLOIDS AND SURFACES. A, PHYSICACHEMICAL AND ENGINEERING ASPECTS, ELSEVIER, AMSTERDAM, NL, vol. 288, no. 1-3, 5 October 2006 (2006-10-05), pages 52-61, XP005606982 ISSN: 0927-7757

## Description

### FIELD OF THE INVENTION

The present invention relates to a method for preparing a flowing silica composition with or without a functional agent incorporated into the material. The present invention also relates to a flowing silica composition, which can be produced with the method. Furthermore, the present invention relates to a method for regelation of the flowing silica composition. The present invention also relates to protection by encapsulation, to preserve and deliver functional agents in and/or from flowing silica compositions. The present invention further relates to uses of flowing silica compositions.

### BACKGROUND OF THE INVENTION

Silica is a versatile material and it can also be prepared synthetically in many morphologies and it may contain different amounts of water. Silica is also soluble in water and especially the sol-gel derived amorphous silica in more or less porous form can be adjusted to have various dissolution rates in water and water-based solutions (from hours to several months by low temperature processing, ≤ 40 °C), even at body fluid pH (e.g. in body-fluid mimicking solutions), where the solubility of silica is at minimum. One of the most interesting features of silica is its interaction with many living organisms and biomolecules. Certain crystal forms of silica are harmful, as in the case of silicosis, but in amorphous and water-dissolved form silica has been observed to have positive interaction with living organisms and biomolecules. Silica is also quite common in nature and a so called biosilification is often observed, especially in many plants. In addition, one of the most abundant living creatures on earth, diatoms, use a wet synthesis method to prepare a silica "skeleton" to cover its organic part. Diatoms induce synthesis of amorphous silica by extracting the needed soluble silica, silicic acid, from sea water that nucleates and condensates on diatoms.

One of the most studied methods to prepare silica is the sol-gel method. Both the sol-gel process and the resulting silica structure resemble silica structures and processes forming and occurring in nature, both in the biosilicification processes and in geological processes, e.g. formation of opals or silica films forming on rocks. The sol-gel process is done in liquid phase, which makes it potential for many applications, e.g. encapsulation of different functional agents. Sol-gel derived SiO₂ and other SiO₂-based materials are commonly prepared from alkoxides, alkylalkoxides, aminoalkoxides or inorganic silicates that via hydrolysis form partly hydrolysed silica species and/or fully hydrolysed form, silicic acid. Consequent condensation reactions of SiOH containing species lead to formation of larger silica species with increasing amount of siloxane bonds. These silica species oligomerize/polymerise and small particles are formed, turning the reaction solution to a sol. The process can be further controlled either to result in particulate sols, i.e., colloidal silica dispersions, i.e., as syntheses are done in alkaline pH & relatively great amounts of water & alcohol, the colloidal particles grow in size & number and do not aggregate or aggregate only in some extent and the formulation stays in the form of a sol. Acidic silica sols are commonly used to prepare gels that are formed as small nanoscale particles aggregate in solution, aggregates grow in size, collide and finally form a gel. In acidic sols, the pH can also be increased to 5-7 to accelerate condensation after hydrolysis and desired sol aging, which is also common in encapsulation, i.e., due to addition of sensitive additives, e.g. proteins and viruses into a sol. The pH increase may also be accompanied with the addition of extra liquids, like water and alcohols to control the gel formation, e.g. to avoid too fast gel formation. Gels can also be formed from alkaline sols, e.g. by adding salt and/or additional sol and/or other solvents into the sol and/or by pH changes. Reactions (typically at ≤ 40°C) are commonly catalysed or the reactions are steered to desired directions in one or several steps either by mineral acids (e.g. HCl and HNO₃), other acids (e.g, CH₃COOH) or bases (e.g. NaOH or NH₃). The formed gel is then aged (typically at ≤ 40°C), dried (aging and drying often simultaneously) to different water content (typically at ≤ 40 °C) and/or heat-treated (typically at ≤ 700°C) to desired form resulting typically in amorphous and porous SiO₂. The last step, heat treatment at elevated temperatures (50-700°C) is typically skipped if the system contains functional agents that do not tolerate elevated temperature, such as many biologically active agents. The gels that are dried at moderate temperature (typically at ≤ 40°C) are generally called xerogels (<Gr. xero=dry), but in spite of their name, they often contain more or less water. The silica gels containing substantial amounts of water, e.g. 30-95 %, are sometimes called silica hydrogels, but the solid, gel-like structure is still dominating the physical appearance.

Amorphous silica made by the sol-gel method is known to result in nanoscale porous structure with varying amount of hydroxyl groups on surface. Amorphous sol-gel derived silica has been observed to have specific interaction with living organisms and many biomolecules. It is known to be biocompatible, (e.g. acceptable response observed in tissue) and known to dissolve in the living tissue as well as in solutions simulating the inorganic part of real human body fluid, e.g. in a water solution buffered to pH 7.4 at 37°C with or without inorganic salts found in real body fluids. Consequently, sol-gel derived silica and other amorphous silica-based materials are also used as such in biomaterials applications and tissue engineering. Due to possibility for easy encapsulation of different molecules and other active or functional agents by adding them into the reacting sol in liquid phase, silica has also been used as drug delivery device for traditional small-molecule drugs and different biologically and therapeutically active agents, such as proteins and viral vectors. Due to typical porous structure, it is also possible to absorb molecules into a ready-made silica structure.

Encapsulation can also be utilized in many other applications. Many proteins and enzymes are useful in (bio)catalysis or in diagnostic applications as sensors (e.g. antibody-antigen) and they can be encapsulated in sol-gel derived silica, which acts as a carrier material. Also living cells, bacteria and algae can be encapsulated in silica, where they may act as (bio)reactors, e.g. by producing therapeutic proteins or other useful molecules or functional agents, e.g. dyes. Encapsulation and delivery of viruses as viral vector, as well as RNA and DNA are also potential, e.g. in gene therapy. Hence, studies on preservation of the biological activity of proteins and other active agents in silica have been one of the topics of interest in different fields of science. In addition to sensitive agents in different biotechnology-related applications, it is also possible to encapsulate other active molecules, which are usually easier cases with respect to preservation of their activity and functionality, such as antimicrobial agents, fragrances, perfumes, colours & dyes, food colours, food additives, fertilisers, antioxidants, humidifiers, vitamins, explosives, insecticides, herbicides, fungicides and high-price reagents/precursors for chemical reactions.

Molecules and other active agents encapsulated in sol-gel silica are in direct contact with different silica species from the liquid phase to solid-phase dominating gel, where the condensation and pore structure are under continuous development. Quite substantial shrinkage may occur during the aging and drying processes and also chemical reactions, such as condensation, proceed. These processes may also proceed during the storage, which may have crucial effects on the activity of the encapsulated agents. This shrinkage occurs already in the preparation of silica hydrogels and xerogels and it is naturally stronger as additional heat-treatment at higher temperatures is conducted. This has been one of the challenges of the conventional sol-gel derived silica that is used in encapsulation. Separate protecting agents, like sugars, have been used to protect proteins from deactivation, but the protection is commonly weak and partial, because the extensive shrinkage of the structure is still occurring.

Silica prepared by sol-gel method is conventionally processed to three-dimensional structures by casting (e.g. monolithic rods), spinning (fibers), by dipping/draining/ spinning (coatings) or by preparing particles of different size. Particles are commonly prepared either by spray-drying that result in particles or spheres mostly on micrometer scale or by letting the particles grow in size and number in the sol in alkaline conditions, which results in colloidal silica dispersion, i.e., submicron, nanoscale particles in a solution. The liquids in the colloidal dispersion can be evaporated and the formed powder of colloidal particles is typically washed and dried several times. Particles are sometimes prepared also by grinding, e.g. monoliths to desired size. All the conventional sol-gel processing methods involve a step, where the structure is dried and/or heat-treated to some extent and the amount of solutions/solvents like water and alcohols are more or less diminishing.

In prior art, the sol-gel derived silica-based materials are widely studied and used as delivery matrix in different morphologies, such as monoliths, coatings and films, fibres, particles of different size and for different functional agents. The functional agents are often drugs and other therapeutic agents (such as proteins, viral vectors and cells), but also other biologically active agents, such as cosmetic agents. Also other functional agents, such as dyes or agents that produce dyes have been encapsulated and optionally delivered. Sol-gel derived silica is not always used for delivery, but for encapsulation only, e.g. as a support material for different functional agents, e.g. for enzymes and other proteins that are used in biocatalysis and for sensor applications.

However, in all these cases, the produced silica is processed to a solid, three-dimensional form, e.g. to "glasses, "xerogels", "hydrogels", "gel oxides" or "ceramics" that are, e.g. in the form of monoliths, coatings, films, fibres or particles. In other words, the processing includes always at least the formation of xerogel or a hydrogel meaning that after the gel formation, the materials is aged and/or dried to certain extent, typically near room temperature and used in the resulting in three-dimensional form that has some properties that are characteristic for solid materials. The encapsulation of functional agents is commonly done in situ in a sol by mixing the functional agents as long as the liquid phase is still dominating. For many sensitive agents, like proteins and viral vectors, the temperatures have to be kept low, typically at 40°C or below. Small-molecule drugs and other functional agents may tolerate higher temperatures. It is also possible to absorb the functional agents into the ready-made silica, i.e., elevated temperatures can be used in silica processing prior to absorption. Some of the materials prepared and described in prior art may also be used in injection (such as microparticles or powders ground from monoliths), but the preparation includes always more or less extensive aging and/or drying of silica structure, where the resulting solid, three-dimensional form of silica is used and the material is not injectable as such and/or the encapsulation of functional agents does not occur (stable colloidal silica dispersions prepared in alkaline sols).

WO96/03117 by Ducheyne et al. discloses controlled release carriers, where biologically active molecules are incorporated within the matrix of a silica-based glass. Here, silica-based glasses are typically multicomponent glasses, and 100 % SiO₂ is a special case, with a very poor dissolution. The release of the biologically active molecules from the carrier is claimed to occur primarily by diffusion through the pore structure.

WO 97/45367 and WO 01/13924 by Ahola et al. disclose sol-gel derived silica xerogels for controlled release. In WO 97/45367 the preparation of dissolvable oxides (silica xerogels) is carried out by simultaneous gelation and evaporation and results in monolithic xerogels, small particles made by spray-drying or fibres made by drawing. In WO 01/13924 the sol-gel derived formulations vary from silica xerogel to alkyl-substituted silica xerogels that provide controlled and sustained release for encapsulated biologically active agents.

WO 93/04196 by Zink et al. discloses the idea of encapsulating enzymes in a porous transparent glass, prepared with a sol-gel method. The purpose is to immobilize enzymes in the pore structure and thus, the release of the enzymes is to be avoided. These porous, transparent glasses can be used to prepare sensors for qualitatively and quantitatively detecting both organic and inorganic compounds, which react with the entrapped material.

WO 00/50349 by Jokinen et al. and WO 01/40556 by Peltola et al. disclose methods for preparation of sol-gel derived silica fibres. WO 00/50349 discloses a method for adjusting the biodegradation rate of the fibres by controlling the viscosity of the spinning process. WO 01/40556 discloses a method for preparing a bioactive sol-gel derived silica fibre.

WO 2005/082781 by Jokinen et al disclose methods for adjustment of the biodegradation rate of silica xerogel monoliths, microparticles and coatings/thin films based on methods where the original chemical structure silica and connected biodegradation rate obtained by proper precursor ratios can be preserved in spite of induced changes (e.g. forced drying in spray-drying, water addition) prior to gel formation. The resulting silica structures undergo aging and drying resulting in solid, three-dimensional forms of silica, which are used in encapsulation and delivery of biologically active agents.

WO 02/080977 by Koskinen et al. discloses a method for preparation of a biodegradable silica xerogel comprising infecting and/or transfecting viruses.

EP 0680753 by Böttcher et al. discloses different solid composites of metal oxide matrices (among them sol-gel derived silica) and functional agents that have been encapsulated into the matrix and are released from the matrix. The control of the release is related to use of separate controlling and penetration agents in the matrix and the preparation of metal oxides undergoes drying of the matrix prior to use.

WO 2003/034979 and WO 01/80823 by Lapidot et al. disclose microcapsules with a core-shell structure, where the shell is made of sol-gel derived oxides, among them silica, which are used for encapsulation and/or topical delivery of active ingredients. The sol-gel-based preparation results in the formation of solid microcapsules prior to use or further processing.

EP 0336014 by Lovrecich discloses pharmaceutical compositions with controlled release in which the active substance is incorporated. The matrix composite are different oxides, among them silica. The functional agents are absorbed into a ready-made, solid silica powder and the main application is to enhance the drug solubility due to restricted crystallization due to encapsulation in the nanoscale pores.

Cellesi & Tirelli 2006 (Sol-gel syntehesis at neutral pH in W/O microemulsion: A method for enzyme nanoencapsulation in silica gel nanoparticles; Colloids and surfaces. A, Physicochemical and engineering aspects, Esevier, Amsterdam, NL, vol. 288, no. 1-3, 5 October 2006, p. 52-61) discloses a method for enzyme nanoencapsulation in silica gel nanoparticles.

GB 2 103 202 A discloses fabrication of sintered high-silica glasses.

### OBJECTS AND SUMMARY OF THE INVENTION

An object of the present invention is to provide a method for producing a flowing silica composition.

Another object of the present invention is to provide a flowing silica composition.

A further object of the present invention is to provide a silica gel.

A still further object of the present invention is to provide uses of a flowing silica composition
- for the manufacture of a flowing silica gel preparation for administering of a silica composition as such and/or incorporated functional agent,
- for preservation of a functional agent,
- for controlled release of a functional agent, and
- for administering a functional agent or agents for agricultural applications, applications of food production, applications of forestry, pest control and/or environmental applications.

Thus the present invention provides a method according to claim 1.

The present invention also provides a flowing silica composition obtainable by the method of the invention.

The present invention additionally provides a silica gel obtainable by methods of the invention.

The present invention also provides use of a flowing silica composition, according to the claims 15, 16 and 17.

### BRIEF DESCRIPTION OF THE DRAWINGS

Figure 1 illustrates the main features of conventional sol-gel processing and the present invention.
Figure 2 illustrates the use of sols and solutions as such.
Figure 3 illustrates preservation of the biological activity.
Figure 4 illustrates the differences of the products between the present invention and those prepared by the conventional sol-gel processing.
Figure 5 illustrates silica dissolution rates for redispersed flowing silica compositions.
Figure 6 show silica dissolution rates for regelled silica compositions.
Figures 7, 8 and 9 illustrate oscillation measurements for silica compositions before and after the gel point, redispersion and regelation.
Figures 10 shows dynamic viscosities for sols after mixing the precursors.
Figure 11 shows dynamic viscosities for flowing silica compositions after redispersion.
Figure 12 illustrates rheological responses of conventional sol-gel derived materials.
Figure 13 shows a Comparison between the rheological responses between silica composition redispersed before the gel point (sols) and after the gel point (gels).
Figure 14 illustrates the release rates horse radish peroxidise (HRP) encapsulated in silica compositions according to the present invention.

### DETAILED DESCRIPTION OF THE PREFERRED EMBODIMENTS

### Terms

*Gel* should be understood to be a homogeneous mixture of at least one solid phase and one liquid phase, i.e., a colloidal dispersion, where solid phase(s), e.g. silica as such and/or as partly or fully hydrolysed, is the continuous phase and the liquid(s), e.g. water, ethanol and residuals of silica precursors, is homogeneously dispersed in the structure. The gel is viscoelastic and the elastic properties dominate, which is indicated by rheological measurements under small angle oscillatory shear that the elastic modulus, G' is at least 10 times greater than the viscous modulus, G" (G' > 10 x G").

The *sol* should be understood to be a homogeneous mixture of at least one liquid phase and one solid phase, i.e., a colloidal dispersion, where the liquid phase(s), e.g. water, ethanol and residuals of silica precursors, is the continuous phase and the solid phase(s), e.g. colloidal particles of silica and/or as partly or fully hydrolysed silica and/or aggregates of said particles are homogeneously dispersed in the said liquid phase characterized in that the sol has clear flow properties and the liquid phase is dominating.

The term *sol-gel transfer* refers to a process where a sol turns to a gel. The most typical example on a preparation process comprising a sol-gel transfer is as silica and other corresponding materials, such as TiO₂ and ZrO₂ are synthesised from liquid phase precursors, typically alkoxides, alkylalkoxides, aminoalkoxides and inorganic precursors, such as silicate solutions that form after hydrolysis and condensation first particles, which turns the system to a sol, after which the particles aggregate and/or grow in size and the sol turns to a gel either spontaneously (typically in acidic sols) or by induced changes, such as pH change or salt addition (typically in alkaline sols). In the said example on alkoxides and silicate solutions, the sol-gel transfer occurs as a part of the above described longer process, which is often called a *sol-gel process.* The term *sol-gel process* is also commonly used for the preparation of powder of colloidal particles, where the alkaline sols does not actually form a gel, but the liquids in the sol are evaporated resulting in the powder. However, the sol-gel transfer may also occur for ready-made silica powders or other ceramic powders, such as oxide powders, e.g. TiO₂, ZrO₂, Al₂O₃. The powders may have been prepared by any method; also mined powders can be used as such or as modified (e.g. as ground and washed). The sol-gel transfer for the ready-made powders is possible especially for powders that consist of colloidal particles (diameter ca. 5 micrometers or below), i.e., as a colloidal powder is mixed with a liquid, e.g. water it can form a stable suspension, i.e., a sol and it can be further flocculated/coagulated to a gel, e.g. by adjusting pH and/or adding salt and/or other substances that affect the stability, such as other liquids or an additional silica sol.

The term *sol-gel derived silica* refers to silica prepared by the sol-gel process wherein the silica is prepared from liquid phase precursors, such as alkoxides, alkylalkoxides, aminoalkoxides or inorganic silicate solutions, which by hydrolysis and condensation reactions form a sol that turns to a gel or forms a stable sol. The liquids in the stable silica sol can be evaporated, which results in the formation of a powder consisting typically of colloidal silica particles. The resulting gels/particles can be optionally aged, dried and heat-treated and if heat-treated, preferably below 700 °C. The sol-gel derived silica prepared below 700 °C is commonly amorphous. The sols can be let to gel in a mould for form-giving. The sol-gel derived silica can also be prepared by processing to different morphologies by simultaneous gelling, aging, drying and formgiving, e.g. by spray-drying to microparticles, by dip/drain/spin-coating to films, by extrusion to monolithic structures or by spinning to fibres.

*Gel point* shall be understood to mean the time point when the sol that is flowing turns to a gel that is viscoelastic and the elastic properties dominate, which is indicated by rheological measurements under small angle oscillatory shear that the elastic modulus, G' is at least 10 times greater than the viscous modulus, G" (G' > 10 x G"). The viscoelastic properties are commonly measured with a rheometer (a measuring device for determination of the correlation between deformation, shear stress and time) by the oscillatory shear, where shear stresses are small (small angles of deformation). The total resistance in small oscillatory shear is described by the complex modulus (G*). The complex modulus contains two components: 1) elastic modulus, also called storage modulus, G' that describes that material has some elastic properties that are characteristic for a solid material, i.e., the gel system will gain energy from the oscillatory motion as long as the motion does not disrupt the gel structure. This energy is stored in the sample and is described by elastic modulus; 2) viscous modulus, also called loss modulus, G" that describes flow properties, i.e., a system, e.g. a silica sol that will in an oscillatory shear create motion between the ingredients of the sol describing the part of the energy, which is lost as viscous dissipation. As G*=G' a material is called elastic and as G*=G" a material is called viscous. At or near the gel point, the elastic modulus, G' becomes larger than the viscous modulus, G". As G' > G", a viscoelastic material is called semi-solid and correspondingly as G" > G, a viscoelastic material is called semi-liquid. The gel point does not necessarily match exactly with the point where G'=G", because a sol with very high viscosity may have elastic properties although it is still flowing. Hence, the gel point should here be understood to be the silica composition where the elastic modulus becomes at least ten times greater than the viscous modulus during the steep increase of the rheological response occurring typically near the gel point, G' > 10 x G". The magnitude of the elastic and viscous modulus depends on the shear stress, which depends on the applied strain (small angle deformation) and frequency (of the oscillatory shear). The measurements are conducted by ensuring an adequate signal for a specific measuring system, i.e., a strain sweep is commonly done at constant frequencies to find the proper signal for the rheometer system and then the actual measurements are done at constant strain with varying frequency. The varying frequencies give varying elastic and viscous modulus, but if the signal for the rheometer system (commonly expressed as 0-100 %) is on proper level (above 1 %) for all chosen frequencies and the total shear stress does not disrupt the material (is observed, e.g. if the elastic modulus starts to decrease although higher frequencies are applied), the difference between the elastic and viscous modulus remains and the measurement show whether the solid or liquid phase dominates. It is also typical that the elastic modulus increases fast after the gel point if the surrounding conditions are not significantly changed, e.g. 100-700 fold increase in G' within few minutes after the gel point is typical for gels formed from acidic sols near room temperature, e.g. for a R15 sol at pH=2 that turns to a gel (R=water-to-alkoxide molar ratio). In the form of a gel after the defined gel point, the solid state dominates, but the system still contains varying amounts of liquids and the material is typically soft and viscoelastic before drying, and hard and brittle if it is extensively dried. In the form of a sol, the liquid state dominates, but the system contains varying amounts of solid phase(s) and the system is still flowing. Before the gel point it is typical that a steep increase in dynamic viscosity and elastic modulus is observed, which continues to rise after the gel point as the structure is developing.

*Induced gelling, regelling* and *gel formation* refers to the sol-gel transfer that is not spontaneous or that is occurring due to/in connection with a form-giving process. The spontaneous gel formation occurs typically in acidic, e.g. alkoxide- or inorganic silicate solution-based sols. However, in alkaline sols or in sols made from separate powders (consisting of colloidal particles) by adding the powder into a liquid, the gelation does not occur without a separate factor that induces gel formation. The factor may be e.g. addition of salt and/or pH adjustment and/or another sol and/or another liquid and/or temperature change and/or change in pressure (e.g. elevation of the temperature or decrease in pressure resulting in a sudden release of volatile components (e.g. water, alcohol, and/or volatile acid or base)) and/or separately introduced energy (e.g. electromagnetic or acoustic),. The sol-gel transfer may also occur simultaneously with a form-giving process in which sols are used, such as spray-drying to microparticles, extrusion to monolithic structures, dip/drain/spin-coating to films, spinning to fibres, freeze-drying to monolithic structures or casting in mould combined with simultaneous applying of any of the inducing factors.

The term *flowing silica compositions* refers to materials that are prepared from a newly-formed gel by redispersing the gel by adding extra liquid under stirring and the said compositions are flowing. The *flowing silica compositions* are prepared from a gel. It is preferable that the redispersion is done right after the sol-gel transfer in order to avoid the development of the structure (condensation reactions proceed, structure shrinks and the material becomes more and more solid, which is commonly indicated, e.g. by steep increase in the elastic modulus after the sol-gel transfer and the gel point). In the case of sols made from separate powders consisting of colloidal particles, the structure does not develop as fast as it will do if, e.g. if alkoxides or inorganic silicate solutions are used in the typical sol-gel process, but also in that case it is preferable to do the redispersion right after the sol-gel transfer to avoid possible changes in flocculated/coagulated gel structure as a function of time.

*Controlled release* refers to desired release rate in delivery of functional agents from silica compositions. Slow (sustained) release is a common goal in delivery of functional agents, e.g. in medical and veterinary use, but also fast release may be beneficial, e.g. in applications, where the main purpose is to protect encapsulated functional agents, e.g. during storage and the immediate release is desired after the storage as the silica composition is applied to use.

*Rheologically homogeneous* refers to flow properties of the flowing and injectable silica composition, which can be injected through a needle, preferably at least through a thin 22G needle, as such or by short (< 30 s) stirring so that the composition stays homogeneous through the whole composition and does not separate to discrete phases. In the context of this application injectable through a specified needle, whether it be a 22G, 23G, 24G, 25G, 26G, 27G, 28G, 29G or 30G needle, a greater G-value is more preferable, refers to that in the conditions defined, i.e. at RT (ca. 25°C), as such or after short (<30 s) stirring, a 400 µl aliquot of the sample can be injected with a 1,0 ml syringe (e.g. BD Plastipak™) using standard injection procedures, i.e. with one steady pressing of the syringe plunger without the use of undue force and without phase separation or blockage of the needles occurring during the injection. Short < 30 s stirring is typically carried out with a vortex mixer. It should be noted that for many preferred embodiments of the invention the silica composition is equally injectable as such as with short < 30 s stirring and short stirring, e.g. as carried out in the examples, has only been carried out in order to standardize procedures.

*Shear Thinning* in the context of this application is a rheological property of a composition. Whenever the shear stress or shear rate of such a composition is altered, the composition will gradually move towards its new equilibrium state and at lower share rates the shear thinning composition is more viscous than newtonian fluid, and at higher shear rates it is less viscous.

*Functional agent* in the context of this application refers to any agent that is desirable to encapsulate and/or to be delivered. Functional agents can be antimicrobial agents, fragrances, perfumes, colours & dyes, food colours, food additives, antioxidants, humidifiers, vitamins, explosives, insecticides, herbicides, fungicides and high-price reagents/precursors for chemical reactions or biologically active agents. *Biologically active agent* in the context of this application refers to any organic or inorganic agent that is biologically active, i.e. it induces a statistically significant biological response in a living tissue, organ or organism. The biologically active agent can be a medicine, peptide, protein, polysaccharide or a polynucleotide, e.g. DNA and RNA. It can be a living or dead cell or tissue, bacteria, algae, a virus, a bacteriophage and a plasmid or a part thereof. It can be an agent for treatment of diseases in therapeutic areas like alimentary/metabolic, blood and clotting, cardiovascular, dermatological, genitourinary, hormonal, immunological, infection, cancer, musculoskeletal, neurological, parasitic, ophthalmic, respiratory and sensory. It can further be for treatment of diseases like osteoporosis, epilepsy, Parkinson's disease, pain and cognitive dysfunction. It can be an agent for the treatment of hormonal dysfunction diseases or hormonal treatment e.g. for contraception, hormonal replacement therapy or treatment with steroidal hormones. It can further be an agent such as an antibiotic or antiviral, anti-inflammatory, neuroprotective, prophylactic vaccine, memory enhancer, analgesic (or analgesic combination), immunosuppressant, antidiabetic or an antiviral. It can be an antiasthmatic, anticonvulsant, antidepressant, antidiabetic, or antineoplastic. It can be an antipsychotic, antispasmodic, anticholinergic, sympathomimetic, antiarrhythmic, antihypertensive, or diuretics. It can be an agent for pain relief or sedation. It can also be a tranquilliser or a drug for cognitive dysfunction. The agent can be in a free acid or base form, a salt or a neutral compound. It can be a peptide, e.g. levodopa; a protein, e.g. a growth factor; or an antibody. It can be a polynucleotide, a soluble ion or a salt.

*Protecting agent or agents* in the context of this application refer to a substance or substances that are useful for protecting and/or enhancing the biological activity of a functional or biologically active agent.

The term *dissolution rate* refers to SiO₂ matrix resorption in TRIS (e.g. Trizma preset Crystals, Sigma) solution buffered at pH 7.4 and 37°C that simulates conditions of body fluids. The TRIS solution is from 0.005 M to 0.05 M. In practice the concentration of TRIS solution is varied according to specific demands of the analysis of a biologically active agent since determination of the release rate of the biologically active agent is typically carried out when the dissolution rate of the matrix is determined. It is common that buffers interfere with many analysis systems that include specific reagents that interact with the analysed target molecule. Such interference is often connected to certain buffer concentration. It should be noted that actual dissolution rates in *in vivo* applications are much slower than those of *in vitro* results due to that concentration gradients *in vivo* differ from those *in vitro.* Accordingly the time for total dissolution are many times longer, typically about 10 times longer, and this should be understood when considering *in vivo* applications.

Determination of the dissolution rate is carried out as follows: The SiO₂ concentration in the TRIS is kept below 30 ppm (to ensure *in sink* conditions; free dissolution of the SiO₂ matrix) during dissolution. The SiO₂ saturation level at pH 7.4 is about 130-150 ppm. When needed, a portion of the dissolution medium is changed to a fresh TRIS buffer in order to keep the SiO₂ concentration below 30 ppm. The dissolution rate is measured from the linear phase of the release curve that is typical after a typical initial deviation (slower or faster phase of release than the linear main part of the release) and before a typical slower phase of the release before the total 100 % SiO₂ dissolution. The linear phase of the release is typically longer than the deviating phases in the beginning or in the end release. The linear phase of the release curve (wt-% dissolved SiO₂/h) can be defined by making a linear regression analysis of the measured release points (wt-% dissolved SiO₂/h). Points of a possible initial deviation phase (slower or faster phase of release than the linear main part of the release) are excluded if the points decrease the linear regression correlation factor (r²) to be < 0.9. The linear phase of the release curve (wt-% dissolved SiO₂/h) can be defined by making a linear regression analysis of measured release points (wt-% dissolved SiO₂/h) with a linear regression correlation factor ≥ 0.9. The total amount (100 wt-%) of SiO₂ is calculated from the theoretical amount of SiO₂ that can be obtained from the sol composition according to the net reaction (e.g. 1 mol of used alkoxide, TEOS corresponds to 1 mol SiO₂).

The term *cell* means any living or dead cell of any organism. Thus cells of e.g. any animal, such as a mammal including a human, plant, bacteria and fungi are included.

*Silica* refers in the context of the present invention preferably to amorphous silica as such, amorphous silica containing water, fully or partly hydrolysed amorphous silica or silica in water-dissolved form, such as silicic acid.

R-values referred to in the application, especially in the examples, are defined by the water-to-alkoxide molar ratio of the recipes. Flowing silica compositions are typically expressed with 2 R-values, e.g., R5-400, where 5 is the initial molar ratio that is used to form the gel and 400 correspond to the total molar water-to-alkoxide ratio after addition of water during the redispersion. However when alcohols or other liquids are comprised in the recipe the R-value is used to calculate the corresponding volume of water and the same volume of alcohol or other liquid is added during redispersion.

### Features of the invention

The present invention is illustrated by comparing its main features to the main features of the conventional sol-gel derived materials. During the conventional sol-gel processing, the silica structures are prepared by turning the sol to a gel or by forming a stable sol. The gel formation may occur spontaneously as, e.g. in acidic silica sols or by forcing and speeding up the process, e.g. by using the sol for fibre spinning, extrusion, dip-coating or spray-drying, where aging and drying occur simultaneously with the gel formation and form-giving. Stable sols are typically formed in alkaline silica sols so that the particles grow in size and number and are not aggregating or only aggregating to some extent, but stay in the form of a sol. The stable sol may also be forced to turn to a gel by adding, e.g. a salt, another sol, another solvent and/or liquid, and/or by pH adjustment. However, the resulting gel structure is different from that of gels prepared from acidic sols. Gels from alkaline sols contain larger particles, they encapsulate additives weaker and they are mechanically weaker. In acidic sols, salt or additional base can be used to further accelerate the otherwise spontaneous process and, e.g. the increase of pH nearer to neutral condition e.g. to pH 5-7 by adding a base is useful and often also compulsory especially when encapsulating biologically active agents, such as proteins, viruses and cells, which are sensitive to too low or high pH. Also changes in conditions through, e.g. evaporation, temperature change, different forms of energy (electromagnetic, acoustic), addition of other liquids, precipitation etc. can be used to accelerate the gel formation. The formed gel structures are commonly let to age and dry, often simultaneously. Aging, drying and optional heat-treatment result in shrinkage until there is a balance with the surrounding conditions. Shrinkage easily destroys the biological activity of encapsulated agents, especially in the case of larger ones, like proteins, RNA, DNA, viruses, algae, bacteria and cells. Colloidal silica sols can be used as such or the liquids are evaporated and after several washing steps and the resulting powder can be remixed, e.g. into water. Optionally, a separate heat-treatment can be done on any morphology, if the encapsulated agents tolerate the temperatures used, but temperatures of 0-40 °C are most common in encapsulation of biologically active agents. The resulting structures can be used as implantable or injectable devices. However, in order to use the conventional materials in injection, additional mixing of the ready-made silica with a liquid can be done.

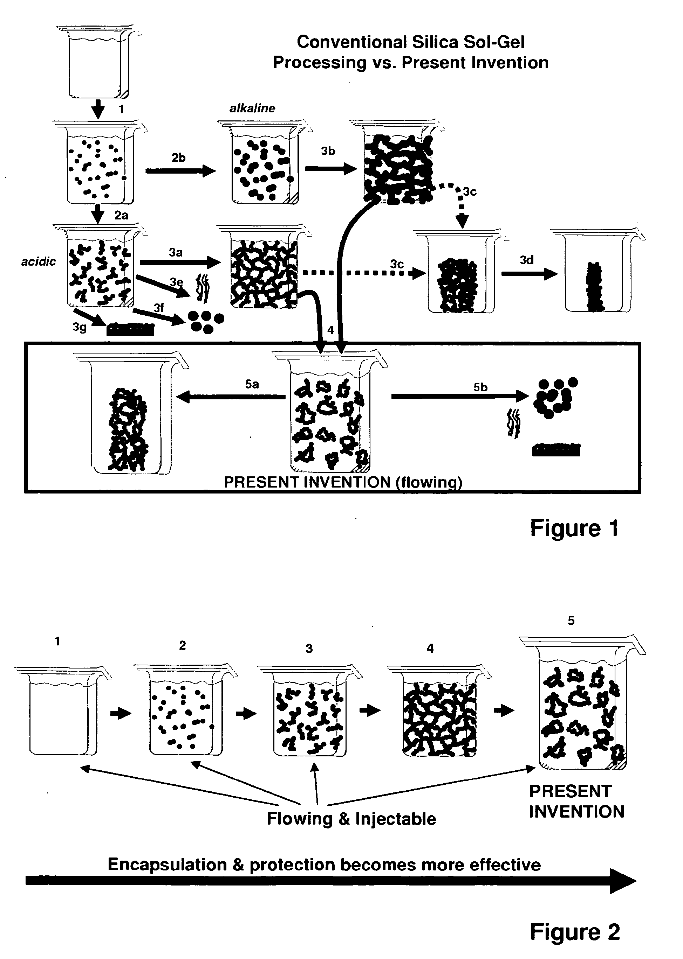

In the present invention, the sol has turned to a gel and the gel is redispersed in a liquid, e.g. water, under stirring soon after the gel formation. The resulting silica gel composition is flowing and injectable and encapsulated agents, such as viruses and proteins retain their biological activity at least for months. The formation of the gel ensures that any functional agent, e.g. a biologically active agent that has been added into to the sol prior to gel formation, becomes effectively encapsulated. The corresponding process for sols, i.e. the dilution of the sols, is also applicable in order to make the sols more stable for injection (to retard gel formation), but the encapsulation effect will not be optimal, not even for sols (consisting of relatively large aggregates) near the gel point, because the added functional agents still have notable possibilities to move in the sol. It is also possible to use ready-made silica powder (or other ceramic powders, such as oxide powders, e.g. TiO₂, ZrO₂, Al₂O₃ etc.) to form a gel by mixing the powder with a liquid, e.g. water and by adjusting, e.g. the pH. This is possible especially for powders that consist of colloidal particles (diameter ca. 5 micrometers or below), e.g. by mixing a colloidal powder with e.g. water to form a stable suspension, i.e. a sol, which can further be flocculated/coagulated to a gel, e.g. by adjusting pH, and/or adding salt and/or other substances that affect the stability, such as other liquids. After gel formation, re-dispersing of the gel by adding liquid under stirring can be done in a similar way as is done for the gels formed, e.g. by hydrolysis and condensation of alkoxides followed by aggregation of formed particles. Some encapsulation of added functional agents can also be achieved by gelling the ready-made powders by adding the functional agents prior to flocculation/coagulation into the sol. It is also possible to add functional agents when re-dispersing, but also in that case the encapsulation is not optimal. After gel formation, i.e., the gel point, i.e. during aging and/or drying of the gel, the structure develosp further, e.g. the material becomes more elastic, it hardens, shrinks etc. and the characteristics of a solid material develop. Due to this structural development during aging and drying, redispersing into a liquid, e.g. water, under stirring becomes more difficult with time and it is preferable to do it right after the gel formation in order to prepare a flowing silica gel composition that can be injected through thin needles in a syringe, e.g. through a so called 26G needle, i.e. a needle with the gauge diameter of 0.45 mm. Depending on conditions, e.g. low temperature, e.g. near 0 °C, and/or "extreme formulations" of silica (e.g. low water-to-precursor ratios that result in high solid contents in the sol) the time point for redispersing after gel point may be relatively long, e.g. several hours or even longer, because the structural development may be slow. However, typical temperatures for preparation of these compositions, e.g. when biologically active agents are used, are from 0 to 40 °C, because both deactivating ice formation and elevated temperatures are to be avoided, especially sensitive agents like proteins, DNA, RNA, viruses, bacteria, algae and cells are incorporated. The main features of conventional sol-gel processing and the present invention are illustrated in Figure 1.

Figure 1 demonstrates principles of conventional sol-gel processing compared with the present invention. **1**: Particles are formed after hydrolysis and condensation of silica precursors; **2a**: In acidic sols the particles form aggregates and reactions proceed spontaneously, the rate being dependent on the precursor and acid concentrations; **2b**: In alkaline sols the particles grow in size and number. Particles do not aggregate or aggregate only to a minor extent and the sol remains stable, i.e. spontaneous gel formation does not occur. **3a**: Aggregates grow in size and number and the sol turns spontaneously to a gel (increase of pH can be used to accelerate gel formation). A mould can be used to cast the sol to desired three-dimensional gel structures, e.g. to rods that can be used as such as implants. **3b**: Colloidal and stable silica dispersion (prepared by any method, either directly from an alkaline sol using e.g. alkoxides or inorganic silicates or a ready-made powder mixed with a liquid) can be gelled by adding salt, another sol, another solvent and/or by pH adjustment and also cast by using a mould. **3c**: In conventional sol-gel processing, the gel is let to age and/or dry at moderate temperatures to a xerogel (<Gr. xero=dry), which can be used as such as e.g. implants. **3d**: In conventional sol-gel processing, the aged and/or dried gel structures can be further heat-treated at elevated temperatures; **3e**: Conventional sol-gel processing; the sol is spun to fibres with simultaneous drying, additional heat-treatment being sometimes used; **3f**: Conventional processing: the sol is spray-dried to microparticles and an additional heat-treatment step is sometimes used. **3g**: Conventional processing: the sol is used to coat a device, e.g. by dipping, spinning or draining; an additional heat-treatment step is common. **4**: Present invention (analogous also for other oxides, e.g. TiO₂ and ZrO₂): the newly-formed gel is re-dispersed (or, e.g. by analogy, dilution of a sol near the gel point containing great amounts of large silica aggregates is re-dispersed) right after gel formation by adding liquid, e.g. water, under stirring to a flowing and injectable silica composition. Gel formation (or presence of great amounts of large aggregates in the sol just before the gel formation) prior to redispersing ensures that added functional agents have a possibility to stay encapsulate. The composition stays flowing and injectable at least for several months; **5a**: Optional for the present invention: The re-dispersed, flowing and injectable silica composition can also be gelled by adding salt and/or an additional portion of another sol with high solid content and/or by adding another solvent and/or by changing the pH or can gel due to an inherent property of the composition to a three-dimensional monolithic form, e.g. in a mould or upon contact with the site of application. Regelling can also be utilised to stimulate the formation of a three-dimensional structure right after injection in tissue, which may be advantageous for controlled release, because the gelled structure is denser than the flowing and injectable composition as such. **5b**: Optional for the present invention: The redispersed flowing and injectable silica composition can be further processed as such or by additional dilution by methods including drying/evaporation of liquids and consequent forced gel formation, e.g. for microparticle preparation by spray-drying, coating of implants by spinning, dipping, draining and corresponding techniques, spinning to fibres or by extrusion to monolithic structures, such as rods.

There are several theoretical possibilities to prepare flowing and injectable formulations by conventional sol-gel processing, but they are not very good with respect to encapsulation, protection and delivery of functional agents. Flowing and injectable formulations can be prepared by conventional sol-gel processing either by dispersing ready-made silica (e.g. spray-dried microparticles or particles ground from silica xerogel monoliths) into a liquid, e.g. into water or into another pharmaceutically accepted liquid, like glycerol or by using the sols or solutions as such and/or by diluting them to retard gel formation. The use of sols and solutions as such is illustrated in Figure 2. The disadvantage of the use of sols and solutions is weak encapsulation and/or dynamics of the structure (turns to a gel). Encapsulation is weak from at least two viewpoints; 1) functional agents can move freely in the sol and only surface reactions and partial encapsulation in larger aggregates is possible and 2) dilution of sols also means that the relative amount of silica, the matrix that should encapsulate functional agents, becomes lower.

In stable alkaline sols including colloidal silica particles and/or weakly aggregated structures, encapsulation of added agents into the silica particle bulk is not practically possible, especially for larger agents like proteins, RNA, DNA, viruses, bacteria, algae and cells, which are of corresponding size or larger than the forming silica particles. In addition, encapsulation of smaller molecules is unlikely and it would disturb the reactions forming colloidal silica particles. The particles grow gradually in size mainly by Ostwald ripening on the particle surface meaning that the same kind of silica aggregates and networks are not present that are present in acidic sols.

Mixing of ready-made silica with liquids for injection has the same restrictions as any silica xerogels or other silica glasses or ceramics, i.e. they undergo heavy structural development and shrinkage of the structure during the process. For example, in a spray-drying process, added functional agents can be added into sol and they become encapsulated in resulting microparticles during spray-drying. These particles can be mixed into liquid, e.g. water, and injected through thin needles, but the biologically active agents are easily deactivated at the elevated temperatures and/or due to heavy shrinkage of the silica structure (deactivating especially sensitive agents like proteins, bacteria, algae, viruses and cells) during drying.

Figure 2 illustrates possible flowing and injectable formulations by conventional methods compared with the present invention. 1: Silica in solution in molecular form, e.g. as silicic acid and/or partly-hydrolysed silica precursors, and/or in oligomerized form (no particles); encapsulation of molecules only theoretical or very weak and partial. The silica species react in practice immediately into particles and accordingly the molecular form is not a real option if alkoxides or corresponding precursors are used. Rapidly forming small nanoscale particles turn the solution into a sol. The molecular form can, however, be formed by dissolving amorphous silica in water, which dissolves into silicic acid; **2**: A sol of colloidal silica particles; encapsulation of molecules only theoretical or very weak and partial, mostly surface reactions possible. The acidic sol prepared, e.g. from alkoxides and inorganic silicates, is also dynamic, i.e. it turns to a gel, which is not flowing and injectable. The dynamics can be reduced by diluting the sol according to same principle as in redispersing. An alkaline sol prepared, e.g. from alkoxides or other sols of colloidal particles, stays in particulate form, but encapsulation is not likely, only surface reactions if functional agents react with SiOH; **3**: A sol, where silica particles have formed aggregates; some encapsulation of molecules possible, though very weak and partial. Larger aggregates are formed in acidic sols that are dynamic and turn to a gel, which is not flowing and injectable. The dynamics can be reduced by diluting the sol according to same principle as in the redispersing. Large-scale aggregation does not occur in alkaline sols and in spite of some aggregation the sols stay stable without gel formation, if no additives are used. Encapsulation is not likely, only surface reactions if functional agents react with SiOH; **4**: Gel point: Silica sol has just turned to a gel; functional agents present in the sol become encapsulated as the gel is formed. 5: Silica gel is re-dispersed to flowing and injectable form with a liquid, e.g. H₂O under stirring; functional agents added into a sol stay encapsulated in solid-dominated nanoscale structures formed at the gel point and preserve their biological activity at least for months.

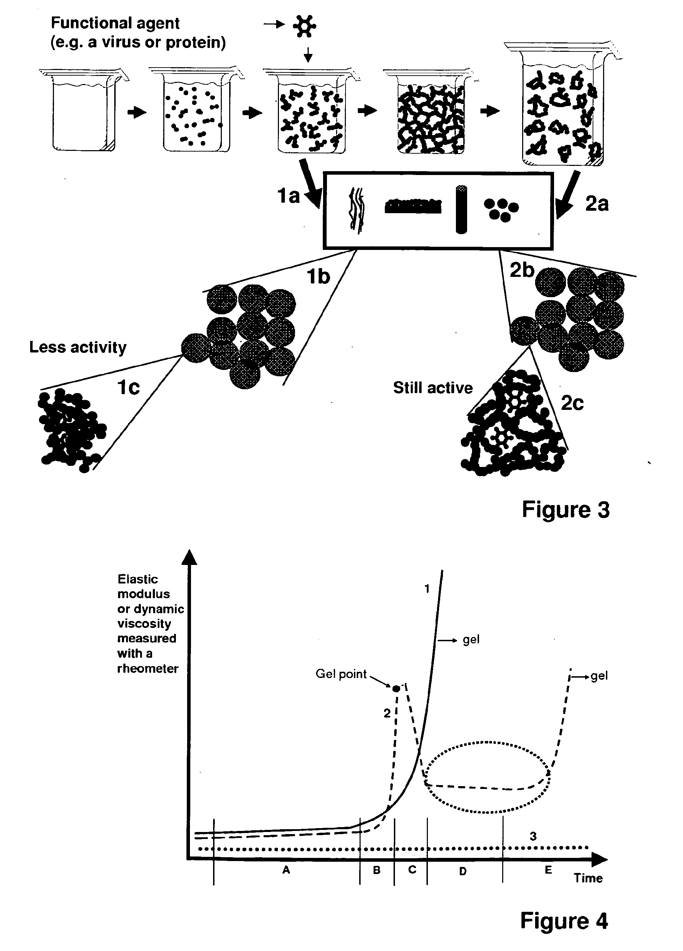

The present invention also provides an option that can be useful in the preparation of conventional silica morphologies, such as monoliths, fibres, particles or coatings/films, especially if sensitive biologically active agents, such proteins, viruses, bacteria, RNA, DNA, algae or cells are encapsulated in silica compositions. As already noted in connection with Figure 1, the redispersed, flowing and injectable silica composition can be used as such and/or by induced changes (e.g. dilution with liquids or additional sols, salt additions, pH adjustments) for preparation of e.g. monoliths, fibres, particles (and further use of particles, e.g. to prepare suspensions) or coatings/films. The potential benefit, better preservation of the biological activity after form-giving, is illustrated in Figure 3. Processing to three-dimensional forms by using the flowing and injectable silica composition differs from conventional processing in that encapsulation has already occurred before form-giving and the encapsulated agents are initially better protected when processed.

Figure 3 shows a schematic comparison of three-dimensional silica structures prepared from conventional sol and redispersed, flowing and injectable silica compositions. **1a**: Form-giving processes in conventional silica sol-gel processing; the sol is processed to monoliths by casting in a mould, spray-dried to particles, to coatings by dipping, spinning, draining or any corresponding method or spun to fibres and the structure shrinks/consolidates heavily during processing to a final form and functional agents added into the sol become encapsulated between the particles. Shrinkage easily destroys the biological activity of sensitive functional agents like proteins and viruses; **1b**. An enlargement of an internal porous structure of silica structures (coatings, monoliths, microparticles, fibres) prepared by a conventional method. **1c**. An further enlargement of the internal porous structure of silica structures prepared by conventional processing, where sensitive encapsulated and biologically active agents easily loose their activity due to shrinking (due to aging, drying, water removal, additional heat-treatment etc.) silica structure. **2**. Present invention, an optional step, i.e. form-giving using the redispersed silica compositions as a "precursor": the added functional agents are already encapsulated before optional additional form-giving after redispersing (casting in moulds, spray-drying, coating, spinning, extrusion etc.) which protects them during shrinkage (some kind of consolidations and shrinkage occur in any form-giving method, also in casting, although the drying that accelerates shrinkage/consolidation can be adjusted, prevented/retarded//accelerated, and leaves more water into the structure that is also beneficial with respect to the preservation of biological activity of sensitive agents like proteins and viruses. **2b**. An enlargement of an internal porous structure of silica structures (coatings, monoliths, microparticles, fibres) prepared from redispersed, flowing and injectable silica composition **2c**. An further enlargement of an internal porous structure of silica structures prepared from the redispersed, flowing and injectable silica composition, where sensitive encapsulated and biologically active agents have better possibility to retain their activity in spite of processing, where shrinking (due to aging, drying, water removal, additional heat-treatment etc.) of silica structure occurs.

The present invention can also be compared with conventional sol-gel processing by rheological measurements. The dynamic viscosity and low-shear oscillation measurements conducted with a rheometer are useful in describing the differences of the products between the present invention and those prepared by the conventional sol-gel processing. These differences are illustrated in Figure 4. Figure 4 shows a schematic picture of the rheological responses of method of the present invention and that of a conventional sol-gel process. **Curve 1**: A typical rheological response (dynamic viscosity, elastic modulus) of a silica sol prepared in acidic conditions (process can be accelerated by increasing pH), with a steep increase in dynamic viscosity/elastic modulus as the number and size of aggregates of silica species starts to approach the gel point and the dynamic viscosity/elastic modulus keeps on increasing after the gel point. Corresponding increase is observed also in alkaline sols as they are gelled by adding salt, another sol, another solvent, and/or by adjusting the pH; **Curve 2**: Present invention, where the gel point is indicated with a black dot; **Curve 3**: Typical rheological response (dynamic viscosity, elastic modulus) of a stable silica sol prepared in alkaline conditions without additives inducing gel formation. **Phase A**: Slow increase of the rheological responses (dynamic viscosity, elastic modulus) in sols after mixing of precursors and before spontaneous (acidic sols) or induced (pH increase, salt addition etc.) increase of dynamic viscosity/elastic modulus; **Phase B**: Steep increase of rheological responses (dynamic viscosity, elastic modulus) near the gel point that occurs spontaneously for sols prepared in acidic conditions (can be also accelerated by increasing pH, e.g. to a level that is suitable for many sensitive biologically active agents, i.e. a pH of 5-7); **Phase C**: Gel point and short aging of the newly-formed gel, most preferably < 2 min during which the dynamic viscosity/elastic modulus may increase) and re-dispersing of the gel by adding liquid under stirring (during which dynamic viscosity/elastic modulus decreases); **Phase D**: Redispersed, flowing and injectable silica composition, which stays injectable at least for several months. After redispersing, the dynamic viscosity/elastic modulus is typically lower than at the gel point. **Phase E**: Optional step, where the redispersed, flowing and injectable silica composition may be gelled again by adding salt, another sol, another solvent and/or liquid, and/or by pH adjustment for casting in a mould or after injection in tissue or by "forced drying" (like spray-drying to microparticles, coating by spinning, dipping or draining or by spinning to fibres). The dynamic viscosity/elastic modulus starts to increase again, the gel is formed and the increase continues after the gel point; **The broken line ellipse** (Phase D & E) describes schematically the time frame wherein the redispersed silica formulation is flowing and injectable. The dynamic viscosity/elastic modulus may either increase or decrease during the storage, but the formulations stay injectable at least for several months. The silica composition stays injectable also for a short time after addition of a salt, another sol, another solvent and/or liquid, and/or after adjustment of pH.

The present invention differs structurally from the silica materials described in prior art. In addition, the present invention introduces a new technical benefit that is not possible with conventional techniques. These new silica formulations are simultaneously flowing and injectable and capable of encapsulating functional agents, even the very sensitive and large ones, like therapeutic proteins, viral vectors, cells, algae, DNA and RNA. The injectable silica formulations provide possibilities to combine easy use, minimal invasion (patient acceptance & conformity with thin needles), encapsulation, and controlled delivery of functional agents. They can also be used as a protecting formulation only, i.e. some formulations are able to encapsulate and protect the functional agents, such as therapeutic drugs and other therapeutic and biologically active agents, e.g. proteins, viruses, bacteria, cells, algae, RNA and DNA, against detrimental conditions, but not necessarily provide a controlled release. The main difference of the present invention compared with conventional sol-gel processing is that extensive structural changes, e.g. shrinkage and evaporation of liquids conventionally occurring during aging, evaporation, drying and heat-treatment phases are avoided. The silica formulations of the present invention contain typically more than 95 % of liquids, water being one of the most potential. During processing, high temperatures are not used (not even instantaneously). The silica formulation of the present invention can be delivered by injection through a thin needle and it encapsulates functional agents of any sizes, from small molecule agent to very large scale agents, like cells and algae. The flowing and injectable silica composition stays injectable for months. The flowing and injectable silica composition is typically shear-thinning and for preferred compositions, the rheological response, e.g. shear rate dependent dynamic viscosity remains almost constant at particular shear rates in spite of high shear stresses. This means that the structure is not strongly affected by the shear (e.g. by injection).

The described redispersing process does not separate the added agents from silica, because encapsulation occurs mostly within the nanoscale structure and re-dispersing the gel by stirring is not able to separate the agents from the silica species. This is demonstrated by the results on biological activity, e.g. viruses stay active for several months, but loose their activity in corresponding conditions in a buffer solution within few days or weeks.

The optional step of the method of the invention, the regelation by adding salt, sol, another solvent and/or liquid, and/or pH adjustment is useful if one wants to enhance controlled release properties of the formulations after injection. The regelated silica compositions are structurally more stable after taking a three-dimensional form and hypothetically also encapsulates better. It also provides a different biodegradation rate that is typically at least partly dependent on the form and size of an object. In the form of a freely flowing, injectable composition, degradation in body fluids is faster and the ability to encapsulate a bit lower. Consequently, a regelated composition provides slower biodegradation rate and thus also slower release for functional agents that are release due to biodegradation, e.g. proteins, viruses, cells, algae and other corresponding agents that are large compared to pores of silica gel formulations.

The flowing silica compositions can be used in many applications where injection or spraying of functional agents is desirable. Injection of the silica composition including a therapeutic agent by a syringe through a thin needle is most potential in medical and veterinary use, but there are also other applications where injection or spraying or corresponding methods of applying can be used, such as spreading of neutralising agents, fertilisers, fodder, manure, insecticides, herbicides and fungicides, which are used, e.g. for environmental purposes, agriculture and forestry.

The flowing silica composition can also be used in combination with reservoir devices for drug delivery but also for other applications. In this context the term reservoir device relates to any closed reservoir or analogous structure with restricted transfer of substance, typically a functional agent, to its surrounding. Reservoir devices for drug delivery have been reviewed by e.g. Lisa Brannon-Peppas in Polymers in Controlled Drug Delivery, Medical Plastics and Biomaterials, Nov 1997, p. 34. Flowing silica composition could be delivered, typically injected, into such a reservoir wherein dissolution of the flowing silica composition would be determined by the conditions within the reservoir and delivery from the reservoir would be determined by the interface of the reservoir separating the reservoir and e.g. a specific tissue that is surrounding it whereto the drug is initially delivered.

Flowing silica compositions and the optional regelled compositions may also be useful in biocatalysis and in sensor applications where the silica compositions act as matrix or support materials and the encapsulated agents, such as proteins like enzymes or antibodies, act as active ingredients.

The use of flowing and injectable silica composition as a precursor for conventional morphologies, such as monoliths, coatings, films, particles of different size and fibres, provides a possibility to better preserve the activity of encapsulated, functional agents.

### Preferred embodiments

According to preferred embodiments of the method of the invention at least one functional agent, preferably biologically active agent, other than the silica as such, is incorporated into said flowing silica composition, by mixing, preferably before the gel point of the sol-gel transfer.

According to especially preferred embodiments of the method of the invention the flowing silica composition is and remains injectable as such or by stirring < 30 s through a 24G, preferably through a 26G, more preferably a 28G and most preferably a 30G needle.

According to many preferred embodiments of the method of the invention adding of the liquid and mixing is carried out within ≤ 10 d, preferably within ≤ 1 d, more preferably within ≤ 10 h, even more preferably within ≤ 3 h and most preferably within ≤ 1 h of reaching the gel point of the sol-gel transfer. According to further preferred embodiments adding of the liquid and mixing is carried out within ≤ 20 min, preferably within ≤ 10 min, more preferably within ≤ 5 min and most preferably within ≤ 2.5 min of reaching the gel point of the sol-gel transfer. Preferred time windows within which adding of the liquid and mixing is to be carried out are recipe dependent and especially temperature dependent. The lower the temperature is, the wider the time window. In general, aging (structural development) of the gel slows down at low temperature and accelerates at higher temperatures. Thus time windows from 1 h to 10 d or even longer are typically feasible using low temperatures in the range of -70 °C to +10 °C , preferably - 20 °C to +5 °C and time windows from 2.5 min, or even less, to 1 h are typically feasible using higher temperatures in the range of +10 °C to +90 °C, preferably +15 °C to +35 °C, more preferably +20 °C to +30 °C and most preferably at RT, i.e. about +25 °C.

Preferred embodiments of the method of the invention comprise the steps of
a) preparing a sol from at least one liquid, preferably water and/or alcohol, and from silica precursors, preferably alkoxides or inorganic silicate solutions, by hydrolysis and condensation of said silica precursors with subsequent particle formation;
b) optionally adding a functional agent, preferably a biologically active agent, or agents, with or without one or more protective agents for said functional agent or agents;
c) letting a sol-gel process reach the gel point; and
d) adding, after having reached gel point of said sol-gel transfer, liquid, preferably water and/or alcohol, into the gel formed by said sol-gel transfer, and said adding being made within a sufficiently short time period after reaching said gel point, said time period depending on temperature and the recipe of the sol-gel transfer, to result, after mixing to follow of said gel and said liquid, in a rheologically homogenous said flowing silica composition, which is and remains injectable as such, or by short stirring < 30 s, through a thin 22G needle.

In further preferred embodiments in step a) the sol is prepared from water, an alkoxide or inorganic silicate solution and optionally a lower alcohol, i.e. an alcohol with ≤ 4 carbons, using an acid or a base as a catalyst, preferably a mineral acid.

In some preferred embodiments of the method said flowing silica composition stored appropriately remains injectable for at least 1 week, preferably 1 month, more preferably 1 year and most preferably 5 years, and said storing preferably comprising storing at ≤+37 °C, more preferably at ≤+25 °C, even more preferably at ≤+15 °C and most preferably at ≤+5 °C.

In many preferred embodiments of the methods of the invention regelling of the flowing silica composition is induced after redispersion. Regelling can be induced in many ways. These include all the alternatives already discussed above for gelling. In some cases it may be beneficial that the same induction methods result in precipitation after injection (in precipitation a phase separation of a silica composition, total or partial, may occur, in (re)gelling the system stays homogeneously in one phase). In some preferred embodiments regelling is induced by adding an agent inducing regelling, preferably selected from the group consisting of a salt, a sol, and a liquid. In other preferred embodiments regelling is induced by adjusting pH. In still further preferred embodiments regelling is carried out by dip, spin, or drain coating; freeze drying; spray drying; fibre spinning; or casting. In these embodiments the flowing silica composition can be a component of a mixture to be (re)gelled. In this context the term "mixture" refers to any mixture comprising a flowing silica composition according to the invention provided that other components of the mixture do not hinder gelling of the mixture. Another silica sol is a particularly preferred other component of the mixture. Depending on the particular application this can result in improved control of dissolution rate of the silica composition as such and/or release of the functional agent optionally incorporated in the composition. When a functional agent is incorporated in the composition also loading, i.e. how much of the functional agent can be successfully incorporated in a defined amount of the composition can be improved.

The invention also relates to embodiments in which regelling after redispersion is followed by further redispersion of the regelled gel. In some particular embodiments it can be advantageous to have several cycles of redispersion and regelling in sequence. Further cycles can, depending on the application, enhance the improvements referred to above.

Preferred flowing silica composition of the invention have at least one functional agent, preferably a biologically active agent, other than the silica gel itself, incorporated into said flowing silica composition, by mixing, preferably before the gel point of the sol-gel transfer.

Especially preferred flowing silica compositions are shear thinning.

In preferred uses for the manufacture of a flowing silica gel for administering to humans or animals especially preferred embodiments have at least one functional agent, preferably biologically active agent, other than the silica as such, is incorporated into said silica composition by mixing, preferably before the gel point of the sol-gel transfer. In further preferred embodiments said use comprises administering selected from the group consisting of oral, buccal, rectal, parenteral, pulmonary, nasal, ocular, intrauterine, vaginal, urethral, topical, dermal, transdermal and surgically implantable administering. In some preferred embodiments the use comprises administering by injection. In still further preferred embodiments regelling of the flowing silica composition is induced in combination with the injecting of the flowing silica composition resulting in regelling of the flowing silica composition following the injection. Preferably induction of regelling is carried out prior to injecting the flowing silica composition.

### EXAMPLES

All silica compositions referred to in the examples to follow not defined to have been prepared from a particular precursor have peen prepared using TEOS (tetraethyl orthosilicate).

### Example 1

### Preparation of re-dispersed (RD) flowing and re-gelated (RG) silica compositions

The silica compositions were prepared using TEOS (= tetraethyl orthosilicate; component A) as the precursor for silica. The initial R= H₂O/TEOS (molar ratio) was varied from R2 to R52.5 and calculated, initial pH in every sample was pH 2 (HNO₃ was used to adjust the pH). After mixing the precursor, the reactions were let to occur at room temperature for 25 minutes prior to pH adjustment of the sol. Prior to actual pH adjustment, all samples, except R52.5-200, were diluted with water to R=H₂O/TEOS=52.5 in order avoid too fast gel formation. After dilution, the pH was raised to 5.5-6.0 by adding 2 M NaOH with vigorous stirring for every sample. The sol turned to a gel, after which the gel was re-dispersed with H₂O under stirring within 0-5 minutes after the gel formation, which changed the molar ratio to R=H₂O/TEOS=200-400. The code for the compositions include the data accordingly, e.g. R52.5-200 means that the initial molar ratio H₂O/TEOS = 52.5 and after re-dispersing it is 200. If the composition is used as such in the flowing form in different characterization methods, it is coded additionally with "RD" (= re-dispersed), e.g. R52.5-200 RD and with "RG" (= re-gelled) if the re-dispersed compositions are additionally re-gelled by adding salt and another sol, e.g. R52.5-200 RG. The regelation of the redispersed flowing silica compositions was done by adding a salt solution [Simulated Body Fluid = body-fluid salts concentrations mimicking (in double salt concentrations) water solution buffered to pH 7.2-7.4 at 37 °C] and a R3 (pH=2) sol into a RD composition in the volume ratio of 1.00/0.75/8.25. The solid contents of the compositions varied between 0.8-3.1 wt-%.

In addition, gels and redispersed silica compositions were prepared from alkaline sols using molar ratios H₂O:TEOS:ethanol=26.7:13.3:60.0 with NH₃ as a catalysts NH₃:TEOS molar ratio being ca. 0.01 yielding to ca. pH=9. The sol was gelled by adding a salt and/or by adjusting the pH to 7. The sol was gelled after 48 hours aging at 40 °C either by adding Ca(NO₃)₂ (to total concentration of 4 x 10⁻⁴ M) or by adjusting the pH to 7. After additions/adjustments, the gels are formed within ca. 20 hours. The redispersion of the formed gels were done by similar way as in the case of the gels derived from the acidic sols resulting in flowing silica composition.

Sodium silicate solution (SiO₂ · NaOH, Sigma-Aldrich) was also used as a precursor to prepare gels that were redispersed to flowing form at room temperature. The contents of the sols are expressed with the R-values (molar water-to-TEOS ratio) via calculation of the corresponding theoretical SiO₂ content for the sodium silicate formulations. The accordingly calculated R-values for sodium silicate formulations varied between R30-50. Redispersions were done in water, which increased R-values to 200-400. Every studied sodium silicate formulation was prepared by the same procedure: The initial pH was adjusted to < 1 with concentrated HNO₃. After slow stirring at room temperature for 25 min, pH was raised to 5-6 by adding 2 M NaOH solution under vigorous stirring. After pH adjustment the sols turned into gels, after which the redispersion of formed gels were done right after the gel point by similar way as in the case where TEOS was used as the precursors. The redispersion of the sodium silicate-derived gels resulted in flowing and injectable silica formulations.

### Example 2

### Silica dissolution rates for redispersed flowing silica compositions

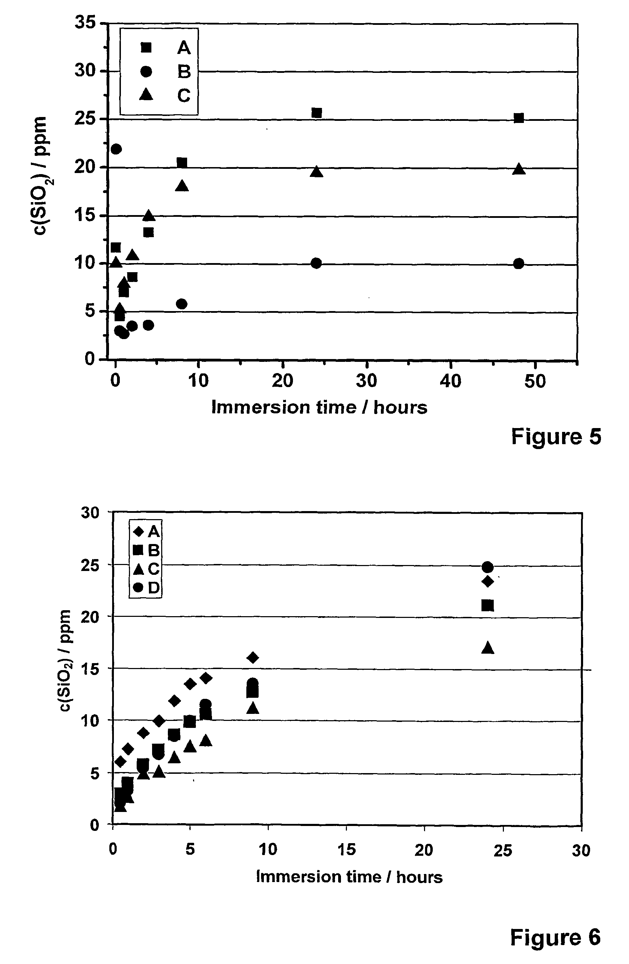

Re-dispersed flowing and injectable silica compositions were studied by immersing them in 0.05 M TRIS buffer solution (pH 7.4, 37°C) for dissolution rate measurements *in sink* conditions [C(SiO₂) < 30 ppm]. The dissolution studies were done in the shaking water bath. The Si concentration of the TRIS buffer at different time points was measured with a spectrophotometer (UV-1601, Shimadzu) analyzing the molybdenum blue complex absorbance at 820 nm. The dissolution rates of the different re-dispersed flowing silica compositions (A= R52.5-200 RD, B= R15-300 RD and C=R5-400 RD) are presented in Figure 5 as cumulative dissolution of SiO₂. The SiO₂ dissolution rates are calculated from the linear part of the graph under ca. 30 ppm (3.32 ppm/h for R52.5-200 RD, 3.29 ppm/h for R15-300 RD and 4.62 ppm/h for R5-400 RD).

### Example 3

### Silica dissolution rates for regelled silica compositions

Redispersed flowing silica compositions (R52.5-200 RD, R30-200 RD, R15-300 RD and R5-400 RD) were stored for 6 months at room temperature (RT) and refrigerator temperature (25°C and 4°C). Redispersed flowing silica compositions that were additionally regelled (A=R52.5-200 RG, B=R30-200 RG, C=R15-300 RG and D=R5-400 RG) were studied after the gel formation by immersing them in 0.05 M TRIS buffer (pH 7.4, 37 °C). The details of the re-gelation are presented in Example 1. RG compositions are made from the corresponding stored RD compositions after the given storage times. The dissolution studies were done in the shaking water bath at 37 °C. The Si concentrations at different time points were measured with a spectrophotometer (UV-1601, Shimatzu) analyzing the molybdenum blue complex absorbance at 820 nm. The dissolution for 6 months stored compositions is presented in Figure 6 as cumulative release of SiO₂. The SiO₂ dissolution rates are calculated from linear part of the graph under ca. 30 ppm. The dissolution rates with different storage time at different storage temperatures are presented in the list below as released SiO₂ per time unit (ppm/h). For all the regelled silica compositions, except R5-400 RG, dissolution rates decreased during the 6 months storage at room temperature. For all compositions at refrigerator temperature and for R5-400 RG also at room temperature the dissolution rate first increased and then decreased during the 6 months' storage.

Dissolution rates for different formulations at different temperatures:
R52.5-200 RG
   ○ RT: 1.69 ppm/h (3 months); 1.21 ppm/h (6 months)
   ○ 4 °C: 2.04 ppm/h (3 months); 1.32 ppm/h (6 months)
R30-200 RG
   ○ RT: 1.73 ppm/h (0 months); 1.71 ppm/h (3 months); 1.61 ppm/h (5 months); 1.16 ppm/h (6 months)
   ○ 4°C: 1.90 ppm/h (3 months); 1.90 ppm/h (5 months); 1,37 ppm/h (6 months)
R15-300 RG
   ○ RT: 1.22 ppm/h (0 months); 1.09 ppm/h (5 months); 1.07 ppm/h (6 months)
   ○ 4 °C: 1.32 ppm/h (5 months); 1.20 ppm/h (6 months)
R5-400 RG
   ○ RT: 1.77 ppm/h (0 months); 2.50 ppm/h (5 months); 1.80 ppm/h (6 months)
   ○ 4 °C: 2.65 ppm/h (5 months); 2.06 ppm/h (6 months)

### Example 4

### Oscillation measurements for 3 silica compositions before and after the gel point, redispersion and regelation

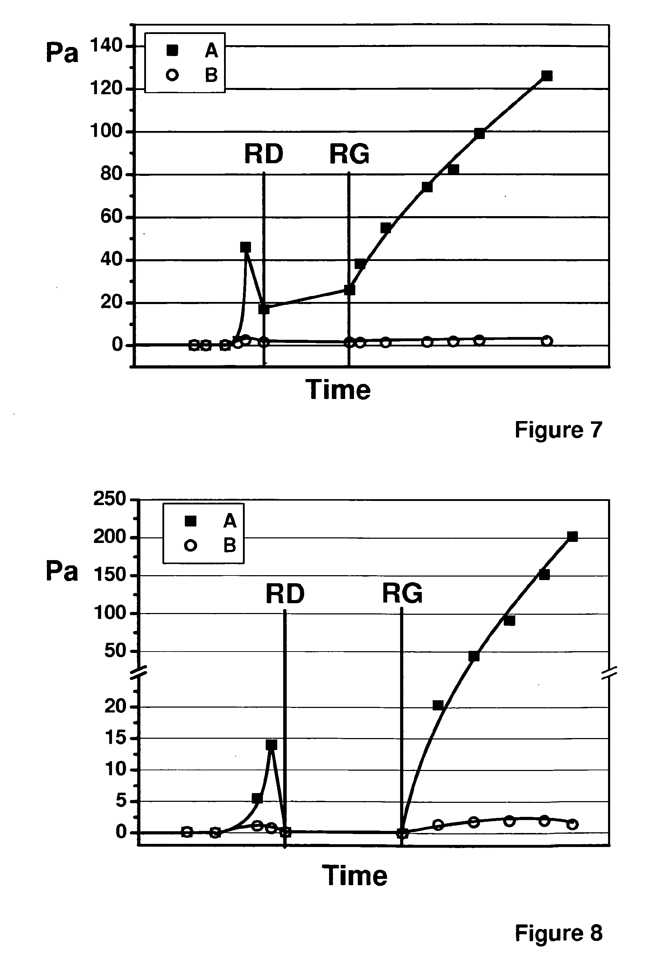

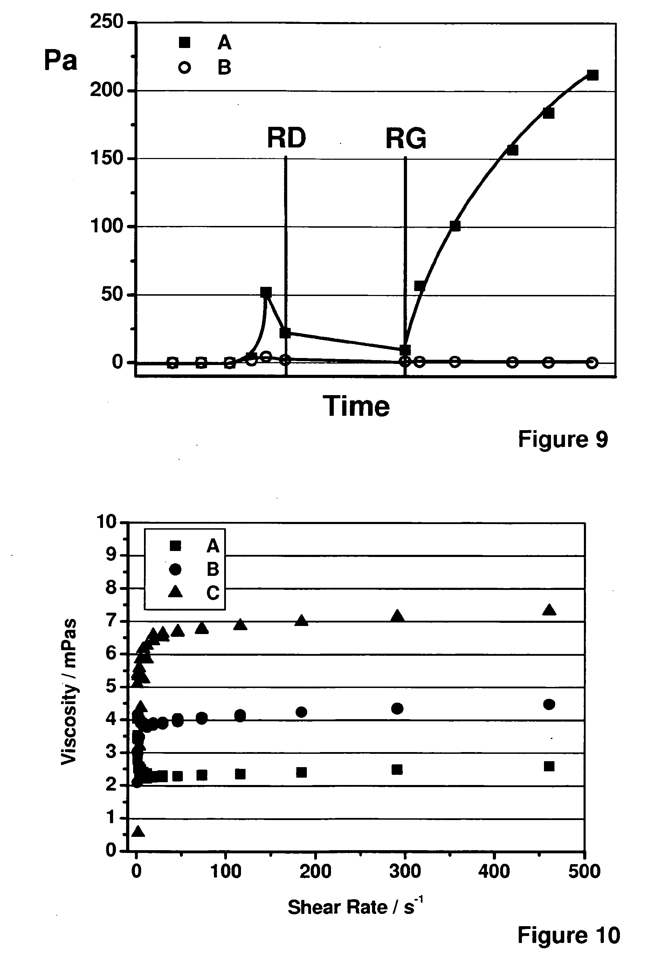

The rheological measurements (done at room temperature in all examples), oscillatory shear by small angle deformation were done for redispersed flowing and regelled silica compositions (R52.5-200 RD & R52.5-200 RG, R15-300 RD & R15-300 RG and R5-400 RD & R5-400 RG) at different phases of the preparation, after mixing the precursors, during the steep increase in the rheological response near the gel point (including also the gel point), right after redispersing, after 1 month's storage at room temperature as redispersed and after addition of salts and another sols (that induce regelation) into the redispersed composition after 1 month's storage. The measurements were done using Bohlin VOR rheometer and measuring system was a concentric, coaxial cylinder sensor system (C 25) ("a bob and a cup" system). The elastic (storage) (G') and the viscous (loss) (G") moduli were determined using oscillatory measuring technique with a constant amplitude of 3 %. Before the gel point and redispersion, the used frequencies were 0.1-2.0 Hz and the torsion element was 0.335 g cm. For measurements of the redispersed flowing silica compositions before and after regelation, the frequency was 0.05-1.0 Hz and the torsion element was 1.94 g cm. The magnitude of the elastic (G') and viscous moduli (G") depends both on the deformation and frequency, but the relative ratio between G' and G" does not vary very much at the same time point. The elastic and viscous moduli of the different RD and RG compositions at the frequency of 0.6 Hz (represents the average) are presented in Figure 7 (R52.5-200 RD/RG), Figure 8 (R15-300 RD/RG) and Figure 9 (R5-400 RD/RG). The formed gels were redispersed as G' (indicated with "A") was 10-15 times greater than G" (indicated with "B"). The typical G' values for the studied compositions varied between 6-60 Pa at/near the gel point.

### Example 5

### Dynamic viscosity for R52.5, R 15 and R5 sols after mixing the precursors

Dynamic viscosity (Figure 10) was measured for *R52.5 sol (A), R15 sol (B) and R5 sol (C)* by Bohlin VOR Rheometer with the concentric, coaxial cylinder sensor system (C 25) ("a bob and a cup" system). Dynamic viscosity was measured at shear rate 0.730 - 461 s⁻¹ (up and down) and the torsion element was 1.94-12.4 g cm.

### Example 6

### Dynamic viscosity for silica compositions (R52.5-200 RD, R 15-300 RD and R5-400 RD) after redispersion

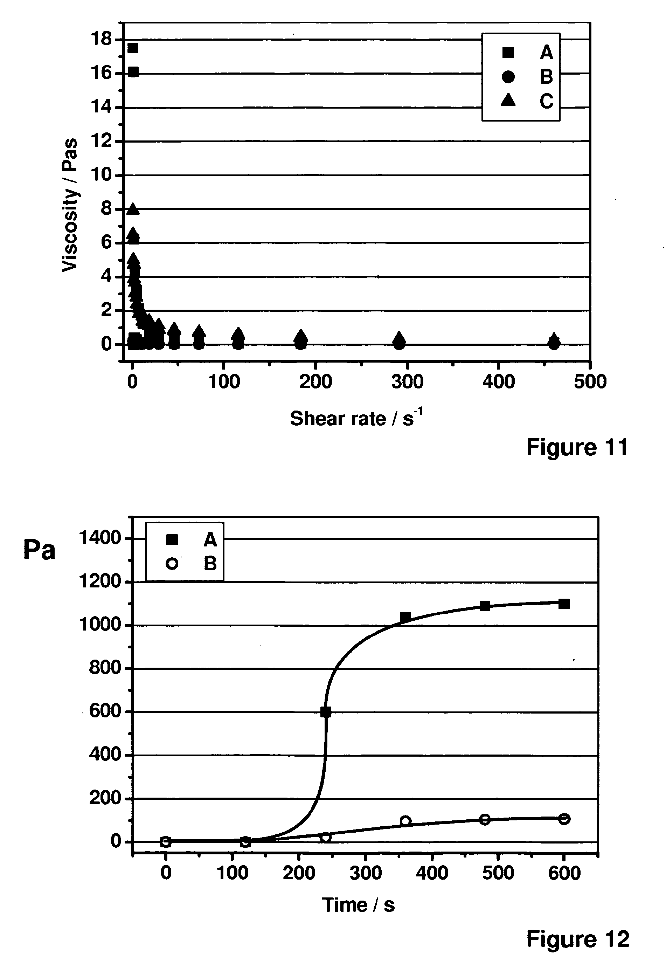

Dynamic viscosity (Figure 11) was measured for *R52.5-200 RD (A), R15-300 RD (B) and R5-400 RD (C)* compositions by Bohlin VOR Rheometer with the concentric, coaxial cylinder sensor system (C 25) ("a bob and a cup" system). Dynamic viscosity was measured at shear rate 0.730 - 461 s⁻¹ (up and down) and the torsion element was 1.94-12.4 g cm. The redispersed, flowing silica compositions show typical shear-thinning behaviour, which is favourable for, e.g. injection. The flowing silica compositions remained shear-thinning after 1 months storage (not shown) and the curve was almost identical both up and down (shear rates). The corresponding results from oscillatory shear are presented in Figures 11, 12 and 13 at the points indicated by RD and RG that shows the situation after storage showing some change as a function of storage time.

### Example 7

### Rheological responses of conventional sol-gel derived materials

The rheological measurements, oscillatory shear by small angle deformation (Figure 12) was measured for conventional sol-gel process for composition R15 (pH 2; process was accelerated after 60 minutes by increasing pH to 5.8 by adding 2 M NaOH) by Bohlin VOR Rheometer with the concentric, coaxial cylinder sensor system (C 25) ("a bob and a cup" system). The used frequencies were 0.1-2.0 Hz and the torsion element was 1.94 g cm and amplitude 3 %. The G' (A) and G" (B) are typical for conventional silica gel preparation from an acidic sol. There is the steep increase before the gel point during which G' becomes clearly dominating and it continues to increase fast after the gel point. Another example on a conventional sol-gel process in alkaline sols (described in example 1; the alkaline sols without induced gelation) was also characterised with the same coaxial cylinder sensor system (C 25) ("a bob and a cup" system). As expected, the oscillatory shear did not give any measurable signal (not shown) for a stable sol consisting of colloidal silica particles. The viscosity measured (not shown) was about 3-4 mPas depending on the shear rate, i.e., not much higher than for water at corresponding conditions (1 mPas at room temperature).

### Example 8

### Comparison between the rheological responses between silica composition redispersed before the gel point (sols) and after the gel point (gels)

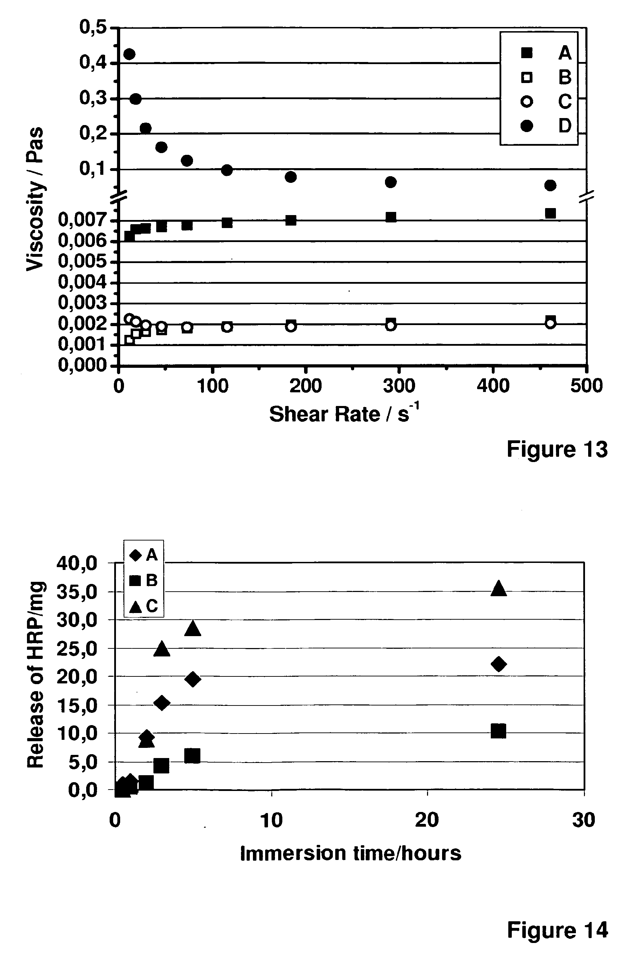

Figure 13 illustrates the difference in the rheological response of the redispersed flowing silica compositions (R5-400 RD ="D") and corresponding sols that are analogically diluted (from R5 to 400 = "B" (first time point) and "C" (second time point) prior to the gel point. In addition, the dynamic viscosity of the R5 sol (="A") after mixing the precursors is also presented. The dynamic viscosity was measured at shear rates of 11.6-461 s⁻¹ (with the same coaxial cylinder sensor system, C 25; "a bob and a cup" system). Dynamic viscosity of R5 sol (A) was 3-6 times higher than the viscosity of the diluted sol (B), which was diluted right after NaOH addition, i.e., it represents a composition, which do not contain larger silica aggregates and the rheological response is still relatively low, even without the dilution. Dynamic viscosity of the redispersed flowing silica composition (R5-400 RD="D"; prepared from the gel right after the gel point within 2 minutes) was 25-50 times higher than dynamic viscosity of the corresponding diluted sol (C) (dilution done short time (some minutes) before the gel point). The dynamic viscosity results show that there is a clear difference between the rheological response between the flowing silica composition prepared by redispersion of the gel and the silica composition prepared by dilution of the corresponding sol.

### Example 9

### Protein encapsulation in flowing silica compositions

A protein (β-galactosidase) was encapsulated into redispersed flowing silica compositions (R52.5-200 RD, R30-200 RD, R25-200 RD, R20-200 RD, R15-200 RD, R10-200 RD, R5-200 RD, R2-200 RD, R15-300 RD and R5-400 RD). Addition of proteins (10 µg/ml silica composition) was done into the sols (R52.5, R30, R25, R20 R15, R10, R5 and R2) after pH adjustment to pH 5.5-6.0 and prior to the gel point. The redispersion was done within 2 minutes after the gel point and the redispersed flowing silica compositions were stained to study the proteins activity as a function of encapsulation time. Encapsulated β-galactosidase was detected from redispersed flowing silica compositions and compared with the corresponding plain redispersed flowing silica compositions (controls) by X-Gal staining method. Each redispersed flowing silica composition was injected through 26G needle (BD Microlance™ 3, 0.45 mm x 16 mm) onto the bottom of 24 well plates well. On the top of the sample, the staining solution (2 mg/ml X-Gal (Eppendorf, 0032006.400, stock 50 mg/ml in N,N-dimethylformamide, Sigma D4551), 0.002 mM MgCl₂ (Sigma, 3143), 0.005 mM K₃Fe(CN)₆ (Riedel de Haën, 31253) and 0.005 mM K₄Fe(CN)₆ (Riedel de Haën) in PBS was added, enough to cover the protein composite. Plate was incubated at 37 °C for 16 hours. After incubation redispersed flowing silica compositions with active β-galactosidase stained blue and the control silica compositions stayed yellow. β-galactosidase remains active at least up to 14 months in the redispersed flowing silica compositions when using TEOS as the precursor.

β-galactosidase was also encapsulated into redispersed flowing silica compositions (R52.5-200 RD, R15-300 RD and R5-400 RD) that were re-gelled according to the method described in example 1 to study the release and encapsulation of the protein from the regelled silica compositions. The regelled silica compositions (R52.5-200 RG, R15-300 RG and R5-400 RG) were immersed in 0.05 M TRIS buffer solution (pH 7.4, 37 °C). The dissolution study was done in the shaking water bath at 37 °C. After two weeks immersion the protein encapsulated RG silica composites were stained with X-Gal method. On the top of the sample the staining solution (2 mg/ml X-Gal (Eppendorf, 0032006.400, stock 50 mg/ml in N,N-dimethylformamide, Sigma D4551), 0.002 mM MgCl₂ (Sigma, 3143), 0.005 mM K₃Fe(CN)₆ (Riedel de Haën, 31253) and 0.005 mM K₄Fe(CN)₆ (Riedel de Haën) in PBS was added, enough to cover the protein composite. Test tubes were incubated at 37 °C for 16 hours. After incubation the R52.5-200 RG, R15-300 RG and R5-400 RG silica compositions were stained and they turned blue showing that there was still active β-galactosidase inside the composite after two weeks dissolution. It shows that β-galactosidase is not significantly diffusing out from the regelled silica compositions.

Two other proteins, horse radish peroxidase (HRP, Sigma-Aldrich) and Lactide dehydrogenase (LDH, Sigma-Aldrich), were encapsulated into redispersed flowing silica compositions (TEOS was used as the precursor) with two different protein concentrations (1% (w/w) and 10 % (w/w) vs. weight of SiO₂). The redispersed flowing silica compositions (R52.5-200 RD, R15-300 RD and R5-400 RD) with both proteins were stored at three different temperature (refrigerator temperature (ca. 4°C), room temperature (ca. 25°C) and 37°C). The enzymatic activity of encapsulated HRP was detected from redispersed flowing silica composition with a spectrophotometer (ThermoLapsystem, Multiscan EX) analyzing the absorbance of yellow colour formed by 3,3',5,5'-tetramethylbentsidine (TMB, Sigma-Aldrich) at 405 nm. Each redispersed flowing silica composition was injected onto bottom of 96-well plates well. On the top of a sample, the TMB solution was added. Plates were incubated at room temperature for 30 min. After incubation the reaction was stopped by adding 0.5 M H₂SO₄. The redispersed flowing silica compositions with HRP were stained as such during the first 5 months and they all showed 100 % activity compared to time point 0. Because the absorbance measured after the reaction was so high, an additional dilution system (1/100000 for flowing silica composition with 10% of HRP and 1/10000 for 1% of HRP)) was used after 5 months' storage. After 6 months' storage as the dilution system was used, no significant decrease was observed in the enzymatic activity of HRP encapsulated in the flowing silica compositions stored at 4°C and 25°C. However, for the flowing silica compositions stored at 37°C, a decrease in HRP activity was observed. The results are presented in the list below as percentage (w/w) of the remaining enzymatic activity compared to the calculated theoretical amount of HRP added into the flowing silica compositions. The enzymatic activity of HRP is well preserved at least for 9 months in the flowing silica compositions stored at 4 °C and room temperature with both 1 % and 10 % of HRP. For the flowing silica compositions stored at 37 °C for 6-9 months, decrease in the enzymatic activity was observed and the decrease was greater in the compositions with 1 % of HRP.

Enzymatic activity of HRP encapsulated in flowing silica compositions as a function of time at different storage temperatures:
R52.5-200 RD (10 % of HRP)
   ○ 37°C: 31 % (172 days), 33% (234 days), 18 % (273 days)
   ○ Room temperature: 82 % (172 days), 86 % (273 days)
   ○ 4°C: 93 % (234 days), 95 % (273 days)
R52.5-200 RD (1 % of HRP)
   ○ 37°C: 7 % (234 days), 1 % (273 days)
   ○ Room temperature: 100 % (234 days), 83 % (273 days)
   ○ 4°C: 100 % (234 days), 100 % (273 days)
R15-300 RD (10 % of HRP)
   ○ 37°C: 69 % (172days), 78 % (234 days), 52 % (273 days)
   ○ Room temperature: 100 % (172 days), 100 % (273 days)
   ○ 4°C: 94 % (234 days), 99 % (273 days)
R15-300 RD (1 % of HRP)
   ○ 37°C: 14 % (234 days), 5 % (273 days)
   ○ Room temperature: 100 % (234 days), 100 % (273 days)
   ○ 4°C: 90 % (234 days), 72 % (273 days)
R5-400 RD (10 % of HRP)
   ○ 37°C: 91 % (172 days), 78% (234 days), 38 % (273 days)
   ○ Room temperature: 100 % (172 days), 100 % (273 days)
   ○ 4°C: 100 % (234 days), 100 % (273days)
R5-400 RD (1 % of HRP)
   ○ 37°C: 19 % (234 days), 8 % (273 days)
   ○ Room temperature: 100 % (234 days), 72 % (273 days)
   ○ 4°C: 100 % (234 days), 100 % (273days)

HRP protein was also encapsulated in to redispersed flowing silica compositions (R30-400 RD, R40-400 RD and R50-400RD) which were prepared using sodium silicate (Sigma-Aldrich) according to the method described in Example 1. The redispersed flowing silica compositions with 10 % protein (w/w compared to m(SiO₂)) were stored at three different temperatures (4 °C, 25 °C and 37 °C). After 3 months' storage the enzymatic activity of the encapsulated HRP was detected from redispersed flowing silica composition with spectrophotometer (ThermoLapsystem, Multiscan EX) analyzing the absorbance of yellow color formed by TMB (Sigma-Aldrich) at 405 nm. The same dilution system was used as described above. The results are presented in the list below as percentage (w/w) of the remaining enzymatic activity compared to the calculated theoretical amount of HRP added into the flowing silica compositions.
R30-400 RD
   ○ 4°C: 27 % (w/w)
   ○ 25°C: 32 % (w/w)
   ○ 37°C: 24 % (w/w)
R40-400 RD
   ○ 4°C: 2 % (w/w)
   ○ 25°C: 5 % (w/w)
   ○ 37°C: 5 % (w/w)
R50-400 RD
   ○ 4°C: 72 % (w/w)
   ○ 25°C: 72 % (w/w)
   ○ 37°C: 17 % (w/w)

The enzymatic activity of encapsulated LDH was detected from redispersed flowing silica composition (TEOS was used as the precursor) by spectrophotometer (ThermoLapsystem, Multiscan EX) at 450 nm and 690 nm. Each studied redispersed flowing silica composition with LDH was injected on to the bottom of 96-well plates well. On the top of sample the staining solution (equivalent amounts of LDH substrate, LDH dye and LDH cofactor). The plate was covered from light and incubated 30 min at room temperature. After incubation the reaction was stopped by adding 1 M HCl. LDH remains active at least up to 7 months with both LDH concentrations at the all studied temperatures (4 °C, room temperature, 37 °C).

The redispersed flowing silica compositions were also used as such in the preparation of microparticles by spray-drying (with a mini spray dryer B-191, Büchi Labortechnik AG, Switzerland; inlet temperature was 80°C, air flow 700 l/h, aspiration 95 %, pump 10 %, resulting microparticles collected into a vessel cooled with an ice bath, spray-nozzle was cooled with running tap water at ca. 5-8°C) and compared with the corresponding sols for preservation of biological activity of encapsulated β-galactosidase. It was observed that some redispersed flowing silica compositions (R15-200 RD, R20-200 RD, R20-400 RD; cyclodextrin was optionally used as a protecting agent and added into the sols (R15, R20) prior to gel formation) preserved the activity of β-galactosidase in resulting microparticles to some extent, which was characterised with the method described above. Activity was observed both with and without the protecting agent. Corresponding preservation of the activity was not observed for microparticles prepared by conventional methods from silica sols in corresponding conditions and spray-drying parameters.

### Example 10

### Virus activity in silica compositions

Activity of adenoviruses was studied in different silica compositions, in redispersed flowing silica compositions in solution with molecular silica species (silicic acid) and in a sol-gel derived silica sol.

Adenoviruses were encapsulated in redispersed flowing silica compositions (R52.5-200 RD, R30-200 RD, R20-200 RD, R15-200 RD, R5-200 RD, R52.5-300 RD, R30-300 RD, R20-300 RD, R15-300 RD, R5-300 RD, R30-400 RD, R20-400 RD, R15-400 RD and R5-400 RD). Addition of viruses was done into the sols (R52.5, R30, R20 and R5) after pH adjustment to pH 5.5-6.0 and prior to the gel point. The redispersion was done within 2 minutes after the gel point and the redispersed flowing silica compositions were stained to study the adenovirus activity (ability of the viruses to infect/transfect) as a function of encapsulation time. Tests were carried out using 24-well plates (Costar). CRL-2592 (ATCC) cells were grown to nearly confluent state using DMEM (Sigma, D5648) supplemented with iFCS 10 % (v/v), antibiotics and NaHCO₃ 1.5 g/I at cell culture environment (+37°C, 5 % CO₂, humidified atmosphere). Just before the applications the medium was changed into fresh medium (1 ml / well). 200 µl of flowing silica compositions and controls were applied on cells through pipette tip and/or injection needle. There were two duplicates for each sample. After applications, the plates were placed into cell culture environment and cultured for 2-3 days, and then stained. Cells were stained with X-Gal method: For staining the cells were washed two times with phosphate buffered (to 7.4) saline (PBS 137 mM NaCl (Riedel de Haën 31434, 2.7 mM KCI Riedel de Haën 31248, 8.1 mM Na₂HPO₄ Riedel de Haën 30427, 1.5 mM KH₂PO₄ Riedel de Haën 30407). Then they were fixed with 0.25 % glutaraldehyde (25 % glutaraldehyde, sigma (G6257) diluted with water for 5 minutes. Then the cells were again washed three times with PBS and the staining solution (2 mg/ml X-Gal (Eppendorf, 0032006.400, stock 50 mg/ml in N,N-dimethylformamide, sigma D4551), 0.002 mM MgCl₂ (Sigma, 3143), 0.005 mM K₃Fe(CN)₆ (Riedel de Haën, 31253) and 0.005 mM K₄Fe(CN)₆ (Riedel de Haën) in PBS was added, enough to cover the cells, through 0.22 µm syringe filter (Sartorius, 16532). Plates were placed back into cell culture environment o/n. Next day the infected/transfected cells were detected by microscopy. This method shows qualitatively that the viruses released from the flowing silica formulations are able to infect the cells (at least some cells infected/well). The results are summarized in table 1 in the column "Qualitative" by indicating the longest preservation time for the virus activity as encapsulated in the flowing silica formulations. The results show that the activity (ability to infect/transfect) of the adenoviruses is preserved at room temperature in several flowing silica formulations for at least 5-6 months. At 4 °C, there are several formulations, where the activity is preserved for 10-12 months.

Another method, so called TClD₅₀ method was used to determine quantitatively the preservation of adenovirus (AdlacZ216; serotype 5; same viruses as in the qualitative test above) activity (infectivity) in the flowing silica formulations. For the TClD₅₀ method, 293 cells (human embryo kidney cells, Microbix Biosystems) were cultured on 96 well cell culture plates, 10 000 cells / well. DMEM with 2 % iFBS was used as the growth medium. Samples were diluted in a logarithmic manner 0.1; 0.01; 0.001 etc. dilutions. Cells on ten parallel wells were infected with 100 µl/well from the dilution and from all the dilutions the number of infected wells was recorded after 10 days of culture at +37 °C, 5 % CO₂, 95 % moisture. The titer, i.e., the number of infective viruses was calculated by the Kärber (also called Spearmann-Kärber method) statistical method.

The direct results from the TClD₅₀ method are expressed as TClD₅₀/ml, which is 0.7 log higher than the titer expressed by the standard plaque assay (plaque forming units = pfu/ml). The results are converted to pfu/ml (summarized in table 1 in the column "Quantitative"; pfu/ml means pfu in 1 ml of the flowing silica formulation and it is indicated in table 1 as "pfu/ml of silica"), because the original virus stock solutions used in the encapsulation were received with data given in pfu. These quantitative results verify that the virus infectivity is preserved in several flowing silica formulations for at least 5-6 months at room temperature and the most accurately studied formulation, R5-400 RD shows also clear infectivity preservation for at least 11 months at 4 °C and for R52.5-200 RD and R30-400 RD even longer (470 and 419 days, respectively). The calculated initial virus amount was 3.2-3.3 x 10⁸ pfu/ 1 ml of the flowing silica formulation in every formulation in the quantitative study.

To show that the encapsulation in the flowing silica formulations has an effect on the preservation of the infectivity, the adenovirus deactivation in the plain phosphate-buffered saline was also studied with the same TClD₅₀ method. Virus titer was measured at the following time points (pfu/ml) at 2 different temperatures. The ratio of the remaining infectivity is given in the parentheses:
37 °C:
   ○ 0 days: 1,20E+10 (100 %)
   ○ 3 days: 1,80E+08 (1.5 %)
   ○ 7 days: 2,00E+0 (0.17 %)
   ○ 14 days: 7,50E+05 (0.01 %)
   ○ 17 days: 3,80E+04 (0.00 %)
   ○ 32 days: 0.00E+00 (0.00 %)
Room temperature
   ○ 0 days: 1,20E+10 (100 %)
   ○ 17 days: 4,00E+08 (3.33 %)
   ○ 32 days: 7,90E+07 (0.66 %)
   ○ 52 days: 7,90E+06 (0.07 %)

The infectivity of the adenoviruses decreases quite fast in the plain phosphate-buffered saline, which verifies that the encapsulation of the adenoviruses in the flowing silica formulations has a clear effect on the preservation of the infectivity of the viruses.

The solution of molecular silica species, silicic acid was prepared by dissolving a sol-gel derived silica gel (R52.5) in PBS buffer (details above) up to SiO₂ concentration of ca. 130 ppm. The molecular SiO₂ species containing PBS was compared with PBS with respect to the adenovirus activity as function of time. Sample virus dilutions was made by adding 100 µl of adenovirus stock (AdlacZ216, titer 2 x 10¹⁰ pfu/ml) into 10 ml of PBS and another 100 µl into molecular SiO₂ species containing PBS. CRL-2592 (ATCC) were cultured at 96-well plates (Nunc, 167008) (conditions and mediums same as above). Sample virus dilutions were kept at +37 °C. At time points logarithmic dilution series was made from sample virus dilutions: 0) 100 µl of original dilution, 1) 10 µl of 0)-dilution + 90 µl of DMEM (same as above), 2) 10 µl of 1)-dilution + 90 µl of DMEM, and so on. Last dilution was 9). Medium was removed from cells and these prepared dilutions were applied onto cells. 100 µl of fresh DMEM was added and plates were incubated at cell culture environment for 2 days. Then they were stained with above mentioned X-Gal method. After 20 days, the adenoviruses were still active in 0)-, 1)- and 2)-dilutions for the molecular SiO₂ species containing PBS, but there was no significant difference between the molecular SiO₂ species containing PBS and controls (fresh PBS with viruses and PBS with viruses after 20 days).

A sol-gel derived silica sol (R300 pH 2 and prior to virus addition it was increased to pH 6.6) was prepared to final volume of about 10.5 ml. The sol remained flowing throughout the test period. Serotype 5 adenovirus, AdlacZ216 was added (200 µl) into the sol and the final virus concentration was of about 10⁷ pfu/ml. PBS buffer (pH 7.4; details above) solution (control) had the same virus content. Both solutions were kept at cell culture environment and samples were cultured at different time points. Culturing was carried out at 24-well plates (costar) using human skin fibroblasts (HSF) established from punch biopsy obtained from a voluntary healthy male donor (age 27), cultured in supplemented DMEM (details above). The sample volume was 10 µl, except at the first time point (7 d) where 200 µl of PBS was used causing larger infection/transfection. The amount of cell culture medium was 1 ml. Infection/transfection was detected by X-Gal staining method (described above). The sample application was carried out on the confluent cell monolayers, except the PBS-Adenovirus control at 21 days and R300 with viruses at 12 days, where the samples (10 µl) were applied together with the cell suspension, which enhances the infection with HSF cells. Time points: PBS-adenovirus control: 7, 10, 15, 17, 21, 28, 34 days; R300 with viruses: 5, 7, 12, 19, 25, 33 days. By qualitative monitoring, the number of Infected/transfected cells decreased as a function of time. After 28 days, there were single infected cells for PBS-adenovirus control, but after 34 days no infection/transfection could

**Table 1**

| **Formulation** | **Qualitative** | | **Quantitative** | | |
|---|---|---|---|---|---|
| | **RT** | **4 °C** | **37 °C** pfu/ml of silica | **RT** pfu/ml of silica | **4 °C** pfu/ml of silica |
| R52.5-200 RD | 199 d | 363 d | | | 2.2 x 10⁵ (470 d) |
| R30-200 RD | 90 d | 326 d | | | |
| R20-200 RD | 151 d | 32 d | | 1.4 x 10⁵ (260 d) | |
| R15-200 RD | 90 d | 92 d | | | |
| R5-200 RD | 151 d | | | 4.5 x 10⁵ (172 d) | |
| R52.5-300 RD | 90 d | 363 d | | | |
| R30-300 RD | 151 d | 129 d | | | |
| R20-300 RD | 151 d | 197 d | | 2 x 10⁶ (179 d) | |
| R15-300 RD | 151 d | 156 d | 1.3 x 10⁵ (21d), | | |
| | | | 9.9 x 10³ (28 d), | | |
| | | | 5.2 x 10³ (31 d), | 2.1 x 10⁶ (146 d), | |
| | | | 1.2 x 10³ (35d), | 8.8 x 10 (179 d) | |
| | | | 4.1 x 10² (38 d), | | |
| | | | 1 x 10² (68 d). | | |
| R5-300 RD | 151 d | 88 d | | 1.9 x 10⁶ (172 d) | |
| R52.5-400 RD | 151 d | | | 1.4 x 10⁵ (168 d) | |
| R30-400 RD | 151 d | 314 d | 1.1 10² (31 d), | 8.9 x 10⁴ (260 d) | 4.1 x 10⁷ (419 d) |
| | | | x 4.0 x 10² (67 d). | | |
| R20-400 UD | 151 d | 314 d | | 1.1 x 10⁵ (260 d) | |
| R15-400 UD | 90 d | 156 d | | 7 x 10⁴ (179 d) | |
| | | | 5.1 x 10³ (21 d), | | |
| R5-400 UD | 151 d | 326 d | 1.1 x 10³ (28 d), | 1.6 x 10⁶ (172 d) | 2.8 x 10⁷ (8 d), |
| | | | 3.2 x 10² (35 d) | | 1.6 x 10⁷ (335 d) |

| | | | | | |
|---|---|---|---|---|---|
| The calculated initial virus amount was 3.2-3.3 x 10⁸ pfu/ 1 ml of the flowing silica formulation in every formulation in the quantitative study. | | | | | |

be observed. Corresponding results were achieved for R300 silica sols with viruses, after 25 days only single infected/transfected cells could be found, and after 33 days no infections/transfections could be observed.

The virus activity results showed that the redispersed flowing silica compositions preserved the activity of the encapsulated adenoviruses at least for 4 months, but in solution with molecular silica species (silicic acid) and in a sol-gel derived silica sol there was no significant difference between the silica composition and the controls.

### Example 11

### Cell Response of the flowing silica compositions

The cell behaviour in contact with cells (CRL-2592 (ATCC)) was monitored in connection with the virus activity tests. No chemical stress could be seen with microscopic examination when testing redispersed flowing silica compositions (R52.5-200 RD, R15-300 RD and R5-400 RD). Cells grew well covering the whole surface of the plate. Some part of cells could be detached, but this is probably caused by physical of silica species. Cell size was similar as with the negative cell control and no increase in vacuolization could be detected. Cell number was increased (qualitative, visual finding) meaning that cell division was not inhibited. Cells looked normal in shape.

Influence of the redispersed flowing silica compositions (R52.5-200 RD, R15-300 RD and R5-400 RD) on the cell growth was compared to the cell growth in plain 24-well cell culture plates (control) in same conditions. The redispersed flowing silica compositions were placed onto nearly confluent cell layers (4 parallel samples for each silica composition and for the control). No difference in the cell growth (done according to the yellow tetrazolium MTT (3-(4,5-dimethyl-2-thiazyl)-2,5-diphenyl-2H-tetrazolium bromide) protocol) was observed between the flowing silica compositions and the control.

RK13 cells (rabbit kidney cells, CCL-37) were encapsulated in the redispersed flowing silica (R5-400 RD) and in the corresponding regelled silica (R5-400 RG). Addition of RK13 cells (ca. 10⁶ cells) was done into the R5 sol after pH adjustment to 5.5-6.0 and prior to the gel point. Redispersion was done right after gel formation. The redispersed flowing silica composition ( R5-400 RD, 400 µl) and the corresponding regelled composition (R5-400 RG, 400µl) was cultured in DMEM (Sigma, D5648) supplemented with iFCS 10 % (v/v), antibiotics and NaHCO₃ 1.5 g/I at cell culture environment (37 °C, 5 % CO₂, humidified atmosphere). R5-400 RD and R5-400 RG without the cells were studied as controls. Both silica compositions were injected through 26G needle (BD, Microlance^{™} 3, 0.45 mm x 16 mm) onto the bottom of 24 well plates well. On the top of the samples culturing medium (1.0 ml) and staining solution (alamarBlue^{™}, 1/10 total volume) were added. Plate was incubated at cell culture environment (37 °C, 5 % CO₂, humidified atmosphere) for 24 hours. After incubation the colour absorbances were measured by a spectrophotometer and the metabolic activity was calculated from the measured results. 29 % of colour was changed (from blue (oxidized form) to red (reduced form)) with R5-400 RD and 27 % with R5-400 RG indicating viability of the encapsulated cells.

### Example 12

### Follow-up on the injectability of different redispersed flowing silica formulations at different storage temperatures

24 different redispersed (redispersions in water) flowing silica compositions (TEOS-derived) were injected (á 400 µl) with the 1.0 ml syringe (BD Plastipak^{™}) with different sizes of needles. All the redispersed flowing silica compositions were shortly (< 30 s) stirred vigorously before the filling of the syringe. All the injections were conducted at room temperature. After storage in closed vessels at room temperature (RT = ca. 25 °C) or at refrigerator temperature (ca. 4 °C) for at least 9 months, most of the redispersed (redispersed right after the gel formation) flowing silica compositions remained as injectable through the same syringe needles as right after the redispersion (0 months). All the injections could be done according to normal use of syringes with one, steady pressing of the syringe plunger and no extra power was needed. All studied formulations, except R52.5-200RD and R30-200RD, remained stable with respect to injectability through thin needles [25G or thinner (external diameter 0.5 mm or thinner)] up to 9 months of storage at room and refrigerator temperatures. After 9 months of storage, the best redispersed flowing silica compositions could be injected through 30G needles (BD Microlance^{™}3; 0.3 mm x 13 mm).

Injectability is expressed in tables 2A, 2B and 2C by providing the thinnest needle size (BD Microlance^{™} 3) through which the injection (á 400 µl) was easy to conduct (one, steady pressing of the syringe plunger with no extra power) with 1.0 ml syringe (BD Plastipak^{™}) and the formulations remained in one phase, i.e., no phase separation was observed during and after the injection and no blockage of the needles occurred.

27G and 30G needles were taken into regular follow-up after 7 months' storage. Before that the thinnest needle used was 26G. Because many of the formulations can be injected through the thinner needles (27G, 30G) after 8 and 9 months' storage, it is clear that it has also been possible also within 0-7 months. One formulation was studied separately with a new batch at 0 months (R5-400 RD) and it was observed that the formulation could be injected through the 30G needle.

To compare injectability with different syringes, a short study with 2 different syringes was conducted. When redispersed flowing silica composition (R15-400 RD stored for 9 months at room temperature) was injected (1 ml) with a larger 10.0 ml syringe (Terumo® syringe), injection (event itself) took longer and more power was needed for the injection than for the injection of 400 µl with a 1.0 ml syringe (BD Plastipak^{™}). The needle is quite easily blocked up when using a 10.0 ml syringe and some withdrawal of the syringe plunger is needed to empty the syringe totally, but with the 1 ml syringe the injection (400 µl) can be done by one, steady pressing. However, no phase separation was detected either with 10.0 ml or 1 ml syringe.

For re-gelling formulations (RG), before the actual gel formation, no practical difference has been observed in the injectability compared with the redispersed (RD) formulations. For R5-400 RG (redispersion done right after the gel point), the injectability remained identical (30G) with the corresponding redispersed formulation (R5-400 RD) for 5 minutes after addition of the salt solution and R3

**Table 2A**

| **Formulation / Storage time** | **0 month** | | **1 month** | | **2 months** | | **3 months** | |
|---|---|---|---|---|---|---|---|---|
| | RT | 4 °C | RT | 4°C | RT | 4 °C | RT | 4 °C |
| R52.5-200 RD | | | | | 25G | 26G | 23G | 25G |
| R30-200 RD | 26G | | 26G | 26G | 26G | 26G | 25G | 26G |
| R25-200 RD | | | | | 26G | 26G | 25G | 26G |
| R20-200 RD | | | | | 26G | 26G | 26G | 26G |
| R15-200 RD | 26G | | 26G | 26G | 26G | 26G | 26G | 26G |
| R10-200 RD | | | | | 26G | 26G | 26G | 26G |
| R5-200 RD | 26G | | 26G | 26G | 26G | 26G | 26G | 26G |
| R2-200 RD | | | | | 26G | 26G | 26G | 26G |
| R52.5-300 RD | | | | | 26G | 26G | 25G | 26G |
| R30-300 RD | 26G | | 26G | 26G | 26G | 26G | 26G | 26G |
| R25-300 RD | | | | | 26G | 26G | 26G | 26G |
| R20-300 RD | | | | | 26G | 26G | 26G | 26G |
| R15-300 RD | 26G | | 26G | 26G | 26G | 26G | 26G | 26G |
| R10-300 RD | | | | | 26G | 26G | 26G | 26G |
| R5-300 RD | 26G | | 26G | 26G | 26G | 26G | 26G | 26G |
| R2-300 RD | | | | | 26G | 26G | 26G | 26G |
| R52.5-400 RD | | | | | 26G | 26G | 26G | 26G |
| R30-400 RD | 26G | | 26G | 26G | 26G | 26G | 26G | 26G |
| R25-400 RD | | | | | 26G | 26G | 26G | 26G |
| R20-400 RD | | | | | 26G | 26G | 26G | 26G |
| R15-400 RD | 26G | | 26G | 26G | 26G | 26G | 26G | 26G |
| R10-400 RD | | | | | 26G | 26G | 26G | 26G |
| R5-400 RD | 26-30G* | | 26G | 26G | 26G | 26G | 26G | 26G |
| R2-400 RD | | | | | 26G | 26G | 26G | 26G |
| *separate batch later for 30G | | | | | | | | |

sol. The same (injection with 30G) was observed for R5-400 RG made from R5-400 RD that was stored for 5 and 9 months both at room temperature and at 4 °C. After the actual gel formation, injectability did not worsen immediately. All of the studied R5-400 RG formulations (stored for 0, 5 and 9 months both at room temperature and at 4 °C) remained injectable through 30G needles at least for

**Table 2B**

| **Formulation / Storage time** | **4 months** | | **5 months** | | **6 months** | |
|---|---|---|---|---|---|---|
| | RT | 4°C | RT | 4°C | RT | 4°C |
| R52.5-200 RD | 21 G | 25G | 23G | 25G | 23G | 23G |
| R30-200 RD | 23G | 26G | 25G | 26G | 25G | 25G |
| R25-200 RD | 23G | 26G | 25G | 26G | 25G | 26G |
| R20-200 RD | 26G | 26G | 26G | 26G | 25-26G | 26G |
| R15-200 RD | 26G | 26G | 26G | 26G | 25-26G | 26G |
| R10-200 RD | 26G | 26G | 26G | 26G | 26G | 26G |
| R5-200 RD | 26G | 26G | 26G | 26G | 26G | 26G |
| R2-200 RD | 26G | 26G | 26G | 26G | 26G | 26G |
| R52.5-300 RD | 26G | 26G | 26G | 26G | 26G | 26G |
| R30-300 RD | 26G | 26G | 26G | 26G | 26G | 26G |
| R25-300 RD | 26G | 26G | 26G | 26G | 26G | 26G |
| R20-300 RD | 26G | 26G | 26G | 26G | 26G | 26G |
| R15-300 RD | 26G | 26G | 26G | 26G | 26G | 26G |
| R10-300 RD | 26G | 26G | 26G | 26G | 26G | 26G |
| R5-300 RD | 26G | 26G | 26G | 26G | 26G | 26G |
| R2-300 RD | 26G | 26G | 26G | 26G | 26G | 26G |
| R52.5-400 RD | 26G | 26G | 26G | 26G | 26G | 26G |
| R30-400 RD | 26G | 26G | 26G | 26G | 26G | 26G |
| R25-400 RD | 26G | 26G | 26G | 26G | 26G | 26G |
| R20-400 RD | 26G | 26G | 26G | 26G | 26G | 26G |
| R15-400 RD | 26G | 26G | 26G | 26G | 26G | 26G |
| R10-400 RD | 26G | 26G | 26G | 26G | 26G | 26G |
| R5-400 RD | 26G | 26G | 26G | 26G | 26G | 26G |
| R2-400 RD | 26G | 26G | 26G | 26G | 26G | 26G |

10 minutes, after which the re-gelled structure started to clearly worsen injectability.

A follow-up study (same 1 ml syringe and same needles as for TEOS-derived formulations) on 3 different sodium silicate-derived silica formulations (R30-200 RD, R40-200 RD, R50-200 RD) was also conducted. At 0 months as well as after 3 months' storage at room temperature and 4 °C, 26G was thinnest needle in use and all formulations could be injected. At 5 months' storage at room temperature and 4 °C, R30-200 RD and R40-200 RD could be injected trough 30G needle, but R50-200 RD with 27G.
Needle sizes (BD Microlance^{™} 3; external diameter x length):
21 G (0.8 mm x 30 mm); 23G (0.6 mm x 30 mm); 25G (0.5 mm x 25 mm); 26G (0.45 mm x 16 mm); 27G (0.4 mm x 13 mm); 30G (0.3 mm x 13 mm)

**Table 2C**

| **Formulation / Storage time** | **7 months** | | **8 months** | | **9 months** | |
|---|---|---|---|---|---|---|
| | RT | 4°C | RT | 4°C | RT | 4°C |
| R52.5-200 RD | 23G | 23G | 23G | 23G | 23G | 23G |
| R30-200 RD | 25G | 25-26G | 23G | 25G | 23G | 23G |
| R25-200 RD | 25-26G | 25-26G | 25G | 25G | 25G | 25G |
| R20-200 RD | 25-26G | 26G | 25G | 26G | 25G | 25G |
| R15-200 RD | 26G | 26G | 25G | 25-26G | 25G | 25G |
| R10-200 RD | 26G | 26G | 26-27G | 26G | 26-27G | 25-26G |
| R5-200 RD | 26G | 26G | 26-27G | 26-27G | 26-30G | 25-27G |
| R2-200 RD | 26G | 26G | 27-30G | 26-30G | 26G | 26G |
| R52.5-300 RD | 26G | 26G | 27-30G | 27-30G | 26-30G | 27G |
| R30-300 RD | 26G | 26G | 30G | 27-30G | 26-27G | 26-27G |
| R25-300 RD | 26G | 26G | 30G | 27-30G | 30G | 27G |
| R20-300 RD | 26G | 26G | 30G | 27-30G | 30G | 30G |
| R15-300 RD | 26G | 26G | 26-30G | 26-30G | 27-30G | 26-30G |
| R10-300 RD | 26G | 26G | 27-30G | 30G | 30G | 30G |
| R5-300 RD | 26G | 26G | 26-30G | 30G | 26-30G | 26-27G |
| R2-300 RD | 26G | 26G | 25-30G | 25-30G | 26-27G | 26G |
| R52.5-400 RD | 26G | 26G | 30G | 30G | 30G | 27-30G |
| R30-400 RD | 26G | 26G | 30G | 30G | 30G | 27-30G |
| R25-400 RD | 26G | 26G | 30G | 30G | 30G | 26-30G |
| R20-400 RD | 26G | 26G | 30G | 30G | 30G | 27-30G |
| R15-400 RD | 26G | 26G | 30G | 27-30G | 30G | 27-30G |
| R10-400 RD | 26G | 26G | 30G | 30G | 30G | 30G |
| R5-400 RD | 26G | 26G | 30G | 27-30G | 27-30g | 30G |
| R2-400 RD | 26G | 26G | 30G | 30G | 30G | 30G |

### Example 13

### Influence of aging time of gel before redispersion on injectability of redispersed flowing silica formulations

Three different flowing silica formulations were studied for injectability (at room temperature, ca. 25 °C) after different aging times of the gel before the redispersion. A short mixing (≤ 30 s) with a vortex mixer was done every time before the filling of the syringes. All the injection experiments were done using a 1 ml syringe (BD Plastipak^{™}) and by injecting 400 µl. Under the aging, the gels were kept in closed, large test tubes at room temperature (at ca. 25 °C). All the redispersions are made by adding water and the mixing in the redispersion is conducted by using a vortex test tube mixer. The injectability of the redispersed formulations is tested right after the redispersion and after 1 week's storage in the closed test tube at room temperature (at ca. 25 °C). The results show that the aging (at room temperature) time of the gel after the gel point (= before the redispersion) should preferably be shorter than 5 minutes in order to achieve good injectability through thin needles like 27-30G (BD Microlance^{™} 3). For the flowing silica products that have been redispersed after a longer (≥ 5 minutes) gel aging time, the redispersion was clearly harder and already a short, one week's storage time worsened the injectability. For 2 of the formulations in this example (R52.5-200 RD and R5-400 RD) redispersed right after the gel point, the good injectability through thin needles is preserved at least for 9 months at different temperatures, which is shown in more detail in the other example, example 12. The accurate needle dimensions are also given in example 12.

### Formulation 1: R15-200 RD

Redispersion 0 minutes after the gel point: Easy to redisperse, results in homogenous dispersion, no visible particles or lumps, easy injection both with 27G and 30G. The dispersion remains in one phase during and after the injection, no phase separation can be observed. After one week's storage at room temperature as redispersed, the injectability (27G and 30G) works as right after the redispersion.

### Comparative example 13a

Redispersion 2.5 minutes after the gel point: A bit harder to redisperse than the gel redispersed right after the gel point (0 minutes), contains gel-like lumps, but they are and stay homogeneously distributed in the dispersion. The formed dispersion is still easy to inject both with 27G and 30G. The dispersion remains in one phase during and after the injection, no phase separation can be observed. After one week's storage in the closed test tube at room temperature as redispersed, the injectability (27G and 30G) works as right after the redispersion.

Redispersion 5 minutes after the gel point: Even harder to redisperse than the gel redispersed 2.5 minutes after the gel point, contains larger gel-like lumps and the lumps separate from liquid phase by falling onto the bottom. However, the injection is still easy both with 27G and 30G after a short (10-30 s) mixing with a vortex mixer. The dispersion remains in one phase during and after the injection, no phase separation can be observed. After one week's storage in the closed test tube at room temperature as redispersed, the injectability has already worsened; the thinnest needle for the injection was 25G.

Redispersion 10 minutes after the gel point: Even harder to redisperse than the gel redispersed 5 minutes after the gel point. The gel had to be separately broken into larger pieces in order to be able to redisperse it in water using the vortex mixer. The formed dispersion contained large gel particles, which fell quite fast onto the bottom. The 1 ml syringe could not be filled directly, but a larger pipette was needed. The thinnest needle that could be used for the injection was 19G. During and after the injection phase separation was observed. After one week's storage in the closed test tube at room temperature as redispersed, no differences were observed in the injectability.

Redispersion 60 minutes after the gel point: Hard to redisperse, comparable to that observed for the gel redispersed 10 minutes after the gel point. The gel had to be separately broken into larger pieces in order to be able to redisperse it in water using the vortex mixer. Mixing during the redispersion could not break the largest particles, which fell fast on the bottom of the test tube. The 1 ml syringe could not be filled directly, but a pipette with a larger diameter was needed. The thinnest needle that could be used for the injection was 19G. During and after the injection phase separation was observed. First came the liquid phase, after which partly dried gel particles. After one week's storage in the closed test tube at room temperature as redispersed, no differences were observed in the injectability.

Redispersion 24 hours after the gel point: Identical observations for the redispersion, injection and for the behaviour after one week's storage as for the formulation redispersed 60 minutes after the gel point.

The longest time for the other formulations studied, R52.5-200 RD and R5-400 RD, for the gel aging time before the redispersion was 10 minutes, because within that time the injectability is already clearly worsened.

*The observations made at the same conditions and at the same time points (0 minutes, 2.5 minutes, 5 minutes and 10 minutes) as for R 15-200 RD were identical for R52.5-200 RD and R5-400 RD with the following exceptions:*

### Formulation 2: R52.5-200 RD

Redispersion 2.5 minutes after the gel point: As for the R15-200 RD at the same time point, but some phase separation is observed after the redispersion. The injectability was identical with that of R15-200 RD. One weeks' storage was not done.

Redispersion 10 minutes after the gel point: As for R15-200 RD, but the thinnest needle that could be used in the injection was 20G. With 19G needle no phase separation was observed during the injection. One weeks' storage was not done.

### Formulation 3: R5-400 RD

Redispersion 2.5 minutes after the gel point: As for the R15-200 RD at the same time point, but some phase separation is observed after the redispersion. The injectability was identical with that of R15-200 RD. One weeks' storage was not done.

Redispersion 10 minutes after the gel point: As for R15-200 RD, but the thinnest needle that could be used in the injection was 21 G. With 20G needle no phase separation was observed during the injection. One weeks' storage was not done.

### Example 14 useful for understanding the invention

### Encapsulation efficiency of flowing silica compositions

Horse radish peroxidase (HRP, Sigma-Aldrich) protein was encapsulated into redispersed flowing silica compositions (R52.5-200 RD, R15-200 RD and R5-200 RD) with 10 % of HRP (w/w vs SiO₂) and they were further regelled according to the method described in example 1 to study that the protein is really encapsulated and it is released as a function of time. The redispersed flowing silica compositions were stored for 9 months at refrigerator temperature (4 °C) after which the regelling was done as described in example 1. The regelled silica compositions (A=R52.5-200 RG, B=R15-200 RG and C=R5-200 RG) were immersed in 0.05 M TRIS buffer solution (pH 7.4, 37 °C). The dissolution study was done in shaking water bath at 37°C. The enzymatic activity of HRP (as shown in example 9, HRP preserves its activity well at 4 °C in several formulations) released into the TRIS buffer at different time points was measured with spectrophotometer (ThermoLabsystems, Multiscan EX) analyzing the absorbance of yellow colour formed by TMB (Sigma-Aldrich) at 405 nm. The release rates of HRP are presented in figure 14. The release results show that HRP is encapsulated and the release occurs as a function of time (the maximum released amounts in Figure 14 correspond to 35 % (w/w) for A, 16 % (w/w) for B and 55 % (w/w) for C)

HRP protein was also encapsulated into R15 (molar water-to-TEOS ratio=15 & pH=2) monoliths (button) and into redispersed flowing silica composition (R13-62 RD). The redispersed flowing silica composition (R13-62 RD) was then used as a co-precursor with another silica sol (R=8, pH2), which together resulted in total formulation of R15 at pH2. The purpose of the study was to show whether there is a difference in the protein encapsulation and release between a normal R15 monolith and R15 monolith including a redispersed flowing silica composition ("R15-incRD"), where the protein was already encapsulated. The HRP content of both sols was 1 mg / ml sol. Both silica compositions were injected (à 150 µl) on the bottom of 96 well plates well. Monolith formation occurred without pH adjustment and formed monoliths were dried to constant weight at constant environment (40 °C and 40 % humidity). The dried monoliths were immersed in 0.05 M TRIS buffer solution (pH 7.4, 37 °C). The dissolution study was done in a shaking water bath at 37°C. The enzymatic activity of HRP in the TRIS buffer at different time points was measured with spectrophotometer (ThermoLabsystems, Multiscan EX) analyzing the absorbance of yellow colour formed by TMB (Sigma-Aldrich) at 405 nm. After 50 hours of immersion in TRIS, the release results showed that the release rate of the encapsulated HRP was about 10% slower from R15-incRD than from the common R15 monolith.

### Example 15 useful for understanding the invention.

### Regelling times of redispersed flowing silica compositions as a function of storage time

24 different redispersed flowing silica compositions (R varied between 2 and 52.5 after the initial sol formation (TEOS was used as the silica precursor) and between 200 and 400 after the redispersion) were stored at two different temperatures (4 °C and 25 °C) to study the effect of the storing time on the regelling. The redispersed flowing silica compositions were regelled according to the method described in example 1. The regelling times (time until the gel is formed after addition of salts and/or sol into the redispersed flowing silica compositions; all the regelations are done at room temperature) varied between 4-13 minutes at 0 months of storage. As a function of the storage time, there was some difference between the storage at 4 °C and 25 °C and some variation also at both temperatures as a function of time. For all the studied compositions, the regelling times were a bit longer (few minutes) for the compositions stored at 4 °C. After 9 months of aging, the variation in the regelling times had extended a little bit, they varied between 4-35 minutes and the longer times (> 20 minutes) were mainly observed for the compositions stored at 4 °C. Some compositions had a relatively constant regelling times as a function of time, e.g., for R5-400 and R2-200 the regelling times stayed between 5-9 minutes after 1, 3, 5 and 9 months of storage at room temperature. There was also a trend in the R-values, the higher R-value after the initial sol formation, e.g., R52.5-400 had somewhat longer regelling times than compositions with lower R-values (e.g., R5-400) and they varied between 7-15 minutes for R52.5-400 after 3, 5 and 9 months of storage at room temperature.

### Example 16 useful for understanding the invention.

### Size of the silica species in the flowing redispersed silica compositions

Although several different flowing silica compositions can be injected trough thin needles (e.g., 27G-30G) so that they stay homogeneously in one phase during and after the injection, they differ visually from each other. Some formulations contain clearly visible silica lumps, for some compositions visible lumps cannot be detected. For some formulations the lumps fall slowly onto the bottom, for some compositions they stay homogeneously dispersed. One of the formulations, R5-400 RD (by visual observation homogeneous even without stirring, no lumps can be detected) was studied by dynamic light scattering and by light microscope. The size distribution of the particles/aggregates/lumps was found to be broad starting from some tens of nanometers reaching to some tens of micrometers. Even few larger, individual aggregates could be detected. Based on observations with a light microscope, the number of larger aggregates (from some tens of micrometers and larger) seems not to be high.

## Claims

1. A method of producing a flowing silica composition, wherein said method comprises
a) performing a sol-gel transfer and
b) carrying out redispersion to obtain a flowing silica composition that stays homogeneous through the whole composition and does not separate to discret phases, comprising.
i) adding liquid, preferably water and/or alcohol, into the gel formed by said sol-gel transfer, within a sufficiently short time period after reaching said gel point, said time period depending on temperature and the recipe of the sol-gel transfer,
ii) said gel and said liquid, to result in a rheologically homogeneous flowing silica composition, which is and remains injectable, when stored appropriately for at least 1 week as such, or by short stirring < 30 s, through a thin 22G needle, whereby a 400 µl aliquot of the sample can at RT be injected with a 1,0 ml syringe, using standard injection procedures, with one steady pressing of the syringe plunger, without the use of undue force and without phase separation or blockage of the needles occurring during the injection.

2. The method according to claim 1 **characterized in that** at least one functional agent, preferably biologically active agent, other than the silica as such, is incorporated into said flowing silica composition, by mixing, preferably before the gel point of the sol-gel transfer.

3. The method according to claim 1 or 2 **characterized in that** said flowing silica composition is and remains injectable as such or by stirring < 30 s through a 24G, preferably through a 26G, more preferably a 28G and most preferably a 30G needle.

4. The method according to any of claims 1 to 3 comprising the steps of
a) preparing a sol from at least one liquid, preferably water and/or alcohol, and from silica precursors, preferably alkoxides or inorganic silicate solutions, by hydrolysis and condensation of said silica precursors with subsequent particle formation;
b) optionally adding a functional agent, preferably a biologically active agent or agents, with or without one or more protective agents for said functional agent or agents;
c) letting the sol-gel transfer reach the gel point; and
d) adding, after having reached gel point of said sol-gel transfer, liquid, preferably water and/or alcohol, into the gel formed by said sol-gel transfer, and said adding being made within a sufficiently short time period after reaching said gel point, said time period depending on temperature and the recipe of the sol-gel transfer, to result, after mixing to follow of said gel and said liquid, in a rheologically homogenous said flowing silica composition, which is and remains injectable as such, or by short stirring < 30 s, through a thin 22G needle.

5. The method according to claim 4 **characterized in that** in step a) the sol is prepared from water, an alkoxide or inorganic silicate solution and optionally a lower alcohol, i.e. an alcohol with ≤ 4 carbons, using an acid or a base as a catalyst, preferably a mineral acid.

6. The method according to any of preceding claims **characterized in that** said flowing silica composition stored appropriately remains injectable for at least 1 month, preferably 1 year and most preferably 5 years, and said storing preferably comprising storing at ≤+37 °C, more preferably at ≤+25 °C, even more preferably at ≤+15 °C and most preferably at ≤+5 °C.

7. The method according to any of claims 1 to 6 **characterized in that** after redispersion regelling of the flowing silica composition is induced.

8. The method according to claim 7 **characterized in that** regelling is induced by adding an agent inducing regelling, preferably selected from the group consisting of a salt, a sol, and a liquid.

9. The method according to claim 7 **characterized in that** regelling is induced by adjusting pH.

10. The method according to any of claims 7 to 9 **characterized in that** regelling of the silica composition as such or as a component of a mixture is induced by carrying out dip, spin, or drain coating; freeze drying; spray drying; fibre spinning; or casting.

11. A flowing silica composition obtainable by the method of any of claims 1 to 5.

12. The flowing silica composition according to claim 11 **characterized in that** at least one functional agent, preferably a biologically active agent, other than the silica gel itself, is incorporated into said flowing silica composition, by mixing, preferably before the gel point of the sol-gel transfer.

13. The flowing silica composition of claim 11 or 12 **characterized in that** the flowing silica composition is shear thinning.

14. A silica gel obtainable by the method of any of claims 7 to 10, preferably as particles, fibres, a coating, or formed monoliths.

15. Use of a flowing silica composition according to claim 12, for preservation of the functionality of said at least one functional agent.

16. Use of a flowing silica composition according to claim 12 for controlled release of said at least one functional agent.

17. Use of a flowing silica composition according to claim 12 for administering a functional agent or agents for agricultural applications, applications of food production, applications of forestry, pest control and/or environmental applications.

## Patentansprüche

1. Verfahren zur Herstellung einer fließfähigen Siliciumdioxid-Zusammensetzung, wobei das Verfahren umfasst:
a) das Durchführen eines Sol-Gel-Übergangs und
b) das Durchführen einer Redispergierung, um eine fließfähige Siliciumdioxid-Zusammensetzung zu erhalten, die in ihrer Gesamtheit homogen bleibt und sich nicht in getrennte Phasen auftrennt, umfassend i) die Zugabe einer Flüssigkeit, bevorzugt von Wasser und/oder Alkohol, zu dem durch den Sol-Gel-Übergang gebildeten Gel innerhalb eines ausreichend kurzen Zeitraums nach dem Erreichen des Gelierpunkts, wobei dieser Zeitraum von der Temperatur und der Rezeptur des Sol-Gel-Übergangs abhängt, und ii) dass das Gel und die Flüssigkeit eine rheologisch homogene, fließfähige Siliciumdioxid-Zusammensetzung ergeben, die in einem injizierbaren Zustand vorliegt und bei geeigneter Lagerung für mindestens 1 Woche, entweder so, wie sie entnommen wird, oder nach einem kurzen Aufrühren von weniger als 30 sec, injizierbar bleibt, und zwar durch eine dünne 22G-Kanüle, wobei ein Aliquot von 400 ml der Probe bei Raumtemperatur mit einer 1,0 ml Spritze unter Anwendung von standardmäßigen Injektionsverfahren mit einem kontinuierlichen Durchdrücken des Spritzenkolbens ohne übermäßige Kraftanwendung injiziert werden kann, ohne dass es im Verlauf der Injektion zu einer Trennung der Phasen oder zu einer Blockierung der Kanülen kommt.

2. Verfahren nach Anspruch 1, **dadurch gekennzeichnet, dass** außer dem Siliciumdioxid selbst mindestens ein funktioneller Wirkstoff, bevorzugt ein biologisch aktiver Wirkstoff, durch Vermischen, bevorzugt vor dem Gelierpunkt des Sol-Gel-Übergangs, in die Siliciumdioxid-Zusammensetzung eingebracht wird.

3. Verfahren nach Anspruch 1 oder 2, **dadurch gekennzeichnet, dass** die fließfähige Siliciumdioxid-Zusammensetzung entweder von Haus aus oder nach einem kurzen Aufrühren von weniger als 30 sec in einem Zustand vorliegt, in dem sie durch eine 24G-Kanüle, bevorzugt durch eine 26G-Kanüle, mehr bevorzugt durch eine 28G-Kanüle und am meisten bevorzugt durch eine 30G-Kanüle injiziert werden kann, und auch in diesem Zustand verbleibt.

4. Verfahren nach einem der Ansprüche 1 bis 3, umfassend die folgenden Schritte:
a) die Herstellung eines Sols aus mindestens einer Flüssigkeit, bevorzugt aus Wasser und/oder Alkohol, sowie aus Vorläufern von Siliciumdioxid, bevorzugt aus Alkoxiden oder anorganischen Silicatlösungen, mittels Hydrolyse und Kondensation dieser Vorläufer von Siliciumdioxid, wobei es im Anschluss zu einer Ausbildung von Partikeln kommt;
b) gegebenenfalls die Zugabe eines funktionellen Wirkstoffs, bevorzugt eines oder mehrerer biologisch aktiver Wirkstoffe, mit oder ohne einen oder mehrere protektive Wirkstoffe für den einen oder die mehreren funktionellen Wirkstoffe;
c) das Abwarten, bis der Sol-Gel-Übergang den Gelierpunkt erreicht hat; und
d) nach dem Erreichen des Gelierpunkts des vorstehenden Sol-Gel-Übergangs die Zugabe von Flüssigkeit, bevorzugt von Wasser und/oder Alkohol, zu dem durch diesen Sol-Gel-Übergang gebildeten Gel, wobei die Zugabe innerhalb eines ausreichend kurzen Zeitraums nach dem Erreichen des Gelierpunkts erfolgt, wobei dieser Zeitraum von der Temperatur und der Rezeptur des Sol-Gel-Übergangs abhängt, um so nach dem Vermischen des Gels und der Flüssigkeit eine rheologisch homogene fließfähige Siliciumdioxid-Zusammensetzung zu erhalten, die entweder von Haus aus oder nach einem kurzen Aufrühren von weniger als 30 sec in einem Zustand vorliegt, in dem sie durch eine dünne 22G-Kanüle injiziert werden kann, und auch in diesem Zustand verbleibt.

5. Verfahren nach Anspruch 4, **dadurch gekennzeichnet, dass** in Schritt a) das Sol aus Wasser, einem Alkoxid oder einer anorganischen Silicatlösung und gegebenenfalls einem niederen Alkohol, d. h. einem Alkohol mit ≤ 4 Kohlenstoffatomen, unter Verwendung einer Säure oder einer Base, bevorzugt einer Mineralsäure, als Katalysator hergestellt wird.

6. Verfahren nach einem der vorhergehenden Ansprüche, **dadurch gekennzeichnet, dass** die fließfähige Siliciumdioxid-Zusammensetzung bei geeigneter Lagerung für mindestens 1 Monat, bevorzugt 1 Jahr und am meisten bevorzugt 5 Jahre in einem injizierbaren Zustand verbleibt, wobei die Lagerung eine Lagerung bei ≤ +37°C, mehr bevorzugt bei ≤ +25°C, noch mehr bevorzugt bei ≤ +15°C und am meisten bevorzugt bei ≤ +5°C umfasst.

7. Verfahren nach einem der Ansprüche 1 bis 6, **dadurch gekennzeichnet, dass** im Anschluss an die Redispergierung ein erneutes Gelieren der fließfähigen Siliciumdioxid-Zusammensetzung induziert wird.

8. Verfahren nach Anspruch 7, **dadurch gekennzeichnet, dass** das erneute Gelieren durch die Zugabe eines die erneute Gelierung induzierenden Wirkstoffs induziert wird, der bevorzugt ausgewählt ist aus der Gruppe bestehend aus einem Salz, einem Sol und einer Flüssigkeit.

9. Verfahren nach Anspruch 7, **dadurch gekennzeichnet, dass** das erneute Gelieren durch eine Einstellung des pH-Werts induziert wird.

10. Verfahren nach einem der Ansprüche 7 bis 9, **dadurch gekennzeichnet, dass** das erneute Gelieren der Siliciumdioxid-Zusammensetzung an sich oder als Bestandteil eines Gemischs durch Tauch-, Rotations- oder Drainagebeschichtung; Gefriertrocknung; Sprühbeschichtung; Faserspinnen; oder Gießen induziert wird.

11. Fließfähige Siliciumdioxid-Zusammensetzung, die durch das Verfahren nach einem der Ansprüche 1 bis 5 erhalten werden kann.

12. Fließfähige Siliciumdioxid-Zusammensetzung nach Anspruch 11, **dadurch gekennzeichnet, dass** außer dem Siliciumdioxid-Gel selbst mindestens ein funktioneller Wirkstoff, bevorzugt ein biologisch aktiver Wirkstoff, durch Vermischen, bevorzugt vor dem Gelierpunkt des Sol-Gel-Übergangs, in die Siliciumdioxid-Zusammensetzung eingebracht wird.

13. Fließfähige Siliciumdioxid-Zusammensetzung nach Anspruch 11 oder 12, **dadurch gekennzeichnet, dass** die fließfähige Siliciumdioxid-Zusammensetzung strukturviskos ist.

14. Siliciumdioxid-Gel, das durch das Verfahren nach einem der Ansprüche 7 bis 10 erhalten werden kann, bevorzugt in Form von Partikeln, Fasern, einer Beschichtung oder geformten Monolithen.

15. Verwendung einer fließfähigen Siliciumdioxid-Zusammensetzung nach Anspruch 12 zur Konservierung der Funktionalität des mindestens einen funktionellen Wirkstoffs.

16. Verwendung einer fließfähigen Siliciumdioxid-Zusammensetzung nach Anspruch 12 zur kontrollierten Freisetzung des mindestens einen funktionellen Wirkstoffs.

17. Verwendung einer fließfähigen Siliciumdioxid-Zusammensetzung nach Anspruch 12 zur Verabreichung eines oder mehrerer funktioneller Wirkstoffe für agrikulturelle Anwendungen, Anwendungen zur Nahrungsmittelherstellung, forstwirtschaftliche Anwendungen, Anwendungen zur Schädlingsbekämpfung und/oder ökologische Anwendungen.

## Revendications

1. Procédé de production d'une composition de silice apte à s'écouler, dans lequel ledit procédé comprend les étapes consistant à
a) réaliser un transfert sol-gel et
b) effectuer une redispersion pour obtenir une composition de silice apte à s'écouler qui reste homogène sur la composition entière et ne se sépare pas en phases discrètes, comprenant :
i) l'addition d'un liquide, de préférence de l'eau et/ou un alcool, dans le gel formé par ledit transfert sol-gel, dans une période suffisamment courte après avoir atteint ledit point de gel, ladite période dépendant de la température et de la formule du transfert sol-gel,
ii) le mélange dudit gel et dudit liquide, pour conduire à une composition de silice apte à s'écouler rhéologiquement homogène, qui est et reste injectable, lorsqu'elle est stockée de manière appropriée ou au moins une semaine telle quelle, ou par brève agitation < 30 s, à travers une fine aiguille 22 G, moyennant quoi une aliquote de 400 µL de l'échantillon peut être injectée à température ambiante avec une seringue de 1,0 mL, à l'aide de procédures d'injection types, avec un pressage constant du piston de seringue, sans utiliser de force injustifiée et sans qu'une séparation de phase ou un blocage des aiguilles ne se produise pendant l'injection.

2. Procédé selon la revendication 1, **caractérisé en ce qu'**au moins un agent fonctionnel, de préférence un agent biologiquement actif, autre que la silice en tant que telle, est incorporé dans ladite composition de silice apte à s'écouler, par mélange, de préférence avant le point de gel du transfert sol-gel.

3. Procédé selon la revendication 1 ou 2, **caractérisé en ce que** ladite composition de silice apte à s'écouler est et reste injectable en tant que telle ou par agitation < 30 s à travers une aiguille 24 G, de préférence 26 G, de manière davantage préférée 28 G et de manière préférée entre toutes 30 G.

4. Procédé selon l'une quelconque des revendications 1 à 3, comprenant les étapes consistant à
a) préparer un sol à partir d'au moins un liquide, de préférence de l'eau et/ou un alcool, et à partir de précurseurs de silice, de préférence des alcoxydes ou des solutions de silicate inorganiques, par hydrolyse et condensation desdits précurseurs de silice avec formation de particules ultérieures ;
b) ajouter facultativement un agent fonctionnel, de préférence un agent ou des agents biologiquement actifs, avec ou sans un ou plusieurs agents protecteurs pour ledit agent ou lesdits agents fonctionnels ;
c) laisser le transfert sol-gel atteindre le point de gel ; et
d) ajouter, après avoir atteint le point de gel dudit transfert sol-gel, un liquide, de préférence de l'eau et/ou un alcool, dans le gel formé par ledit transfert sol-gel, et ladite addition étant effectuée dans une période suffisamment courte après avoir atteint ledit point de gel, ladite période dépendant de la température et de la formule du transfert sol-gel, pour conduire, après mélange s'en suivant dudit gel et dudit liquide, à ladite composition de silice apte à s'écouler rhéologiquement homogène, qui est et reste injectable en tant que telle, ou par brève agitation < 30 s, à travers une fine aiguille 22 G.

5. Procédé selon la revendication 4, **caractérisé en ce que** dans l'étape a), le sol est préparé à partir d'eau, d'un alcoxyde ou d'une solution de silicate inorganique et facultativement d'un alcool inférieur, c'est-à-dire un alcool avec ≤ 4 atomes de carbone, en utilisant un acide ou une base en tant que catalyseur, de préférence un acide minéral.

6. Procédé selon l'une quelconque des revendications précédentes, **caractérisé en ce que** ladite composition de silice apte à s'écouler stockée adéquatement reste injectable pendant au moins 1 mois, de préférence 1 an et de manière préférée entre toutes 5 ans, et ledit stockage comprenant de préférence un stockage à ≤ +37 °C, de manière davantage préférée à ≤ +25 °C, de manière encore davantage préférée ≤ +15 °C et de manière préférée entre toutes ≤ +5 °C.

7. Procédé selon l'une quelconque des revendications 1 à 6, **caractérisé en ce qu'**après redispersion, une regélification de la composition de silice apte à s'écouler est induite.

8. Procédé selon la revendication 7, **caractérisé en ce que** la regélification est induite en ajoutant un agent induisant une regélification, de préférence choisi dans le groupe constitué par un sel, un sol et un liquide.

9. Procédé selon la revendication 7, **caractérisé en ce que** la regélification est induite en ajustant le pH.

10. Procédé selon l'une quelconque des revendications 7 à 9, **caractérisé en ce que** la regélification de la composition de silice en tant que telle ou comme composant d'un mélange est induite en effectuant un revêtement par immersion, centrifuge ou par drainage ; une lyophilisation ; un séchage par pulvérisation ; un filage de fibre ; ou un coulage.

11. Composition de silice apte à s'écouler pouvant être obtenue par le procédé de l'une quelconque des revendications 1 à 5.

12. Composition de silice apte à s'écouler selon la revendication 11, **caractérisée en ce qu'**au moins un agent fonctionnel, de préférence un agent biologiquement actif, autre que le gel de silice lui-même est incorporé dans ladite composition de silice apte à s'écouler, par mélange, de préférence avant le point de gel du transfert sol-gel.

13. Composition de silice apte à s'écouler selon la revendication 11 ou 12, **caractérisée en ce que** la composition de silice apte à s'écouler est à fluidification par cisaillement.

14. Gel de silice pouvant être obtenu par le procédé de l'une quelconque des revendications 7 à 10, de préférence sous forme de particules, de fibres, de revêtement ou de monolithes formés.

15. Utilisation d'une composition de silice apte à s'écouler selon la revendication 12, pour la préservation de la fonctionnalité dudit au moins un agent fonctionnel.

16. Utilisation d'une composition de silice apte à s'écouler selon la revendication 12, pour la libération contrôlée dudit au moins un agent fonctionnel.

17. Utilisation d'une composition de silice apte à s'écouler selon la revendication 12, pour l'administration d'un agent ou des agents fonctionnels pour des applications agricoles, des applications de production alimentaire, des applications de sylviculture, des applications de lutte contre les nuisibles et/ou environnementales.
